# EUROPÄISCHE PATENTSCHRIFT

(11) **EP 0 671 378 B1**
(45) Veröffentlichungstag und Bekanntmachung des Hinweises auf die Patenterteilung: **29.01.1997**
(21) Anmeldenummer: 95102924.8
(22) Anmeldetag: 02.03.1995
(51) Int. Cl.: C07C 45/58, C07C 49/337, C07C 35/18, C07C 35/17, C07C 29/32, C07D 303/14

(54) **Verfahren zur Herstellung von (1R,4S)-4-Hydroxy-1,2,2-trimethylcyclopentyl-methylketon sowie Derivaten und Steroisomeren dieser Verbindung**
Process for the preparation of (1R,4S)-4-hydroxy-1,2,2-trimethylcyclopentyl-methylketone, its derivatives and stereoisomers
Procédé de préparation de la (1R,4S)-4-hydroxy-1,2,2-triméthylcyclopentyl-méthylcétone, de ses dérivés et stéréoisomères

(30) Priorität: 07.03.1994 DE 4407464
(43) Veröffentlichungstag der Anmeldung: 13.09.1995
(73) Patentinhaber: BASF AKTIENGESELLSCHAFT, 67056 Ludwigshafen (DE)
(72) Erfinder: John, Michael, Dr., D-67061 Ludwigshafen (DE); Rheude, Udo, Dr., D-67166 Otterstadt (DE); Paust, Joachim, Dr., D-67141 Neuhofen (DE); Meyer, Joachim, Dr., D-67133 Maxdorf (DE)

(56) Entgegenhaltungen:
- PATENT ABSTRACTS OF JAPAN, vol. 014, no. 043 (C-0681), 26.Januar 1990 & JP-A-01 275545
- PATENT ABSTRACTS OF JAPAN, vol. 012, no. 283 (C-518), 3.August 1988 & JP-A-63 060948
- HELVETICA CHIMICA ACTA, Bd. 66, Nr. 7, 1983, Seiten 1939-1960, A. RÜTTIMANN et al: "Synthese von (3R,3'S,5'R)-Capsanthin, (3S,5R,3'S,5'R)-Capsorubin, (3'S,5'R)-Kryptocapsin und einigen verwandten Verbindungen. Ein neuer Zugang zu optisch aktiven Fünfring-Carotinoidbaustein durch Hydroborierung"
- J. CHEM. SOC. PERKIN TRANS. I, Bd. 1983, Nr. 7, Seiten 1465-1474, R. D. BOWDEN et al: "Carotenoids and Related Compounds. Part 37. Stereochemistry and Synthesis of Capsorubin"

## Beschreibung

Die Erfindung betrifft ein Verfahren zur Herstellung von (1R,4S)-4-Hydroxy-1,2,2-trimethyl-cyclopentyl-methylketon und O-geschützten Derivaten als essentiellen Vorprodukten für den begehrten roten Farbstoff Capsorubin sowie von Stereoisomeren dieser Verbindung sowie neue Zwischenprodukte der allgemeinen Formeln V und VIII.

Capsorubin ist neben Capsanthin und Kryptocapsin ein Pigment der roten Paprika (Capsicum annuum mit folgender Struktur: 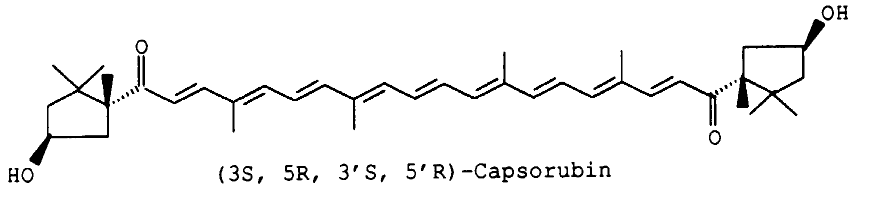 Charakteristisch für dieses Rotpigment ist somit das Vorliegen von Cyclopentanol-Einheiten im Molekül. Die komplizierte Stereochemie der Capsorubinendgruppen ist für Carotinoide ungewöhnlich und wurde erst in den sechziger Jahren endgültig aufgeklärt (vgl. J. Chem. Soc. 1961, Seite 4019 ff.).

Aufgrund der guten Farbwirkung von Capsorubin wurden schon frühzeitig Versuche zu seiner synthetischen Herstellung unternommen. Die erste Synthese von optisch inaktivem Capsorubin gelang Weedon (vgl. Pure Appl. Chem. 14 (1967) Seiten 265-78) in einer Sequenz von 8 Reaktionsstufen, wobei der letzte Schritt aus einer Aldolkondensation von 2 Äquivalenten racemischem 4-Hydroxy-1,2,2-Trimethyl-cyclopentyl-methylketon mit einem Äquivalent an dem C₂₀-Dialdehyd, Crocetindialdehyd, besteht.

Nachdem der Aufbau eines entsprechenden Cyclopentylcarbonsäureesters mit Chiralitätszentrum in 4 Stufen ausgehend von (+)-Campher (vgl. Acta Chem. Scand. B 28 (1974) Seiten 492-500) sowie die Herstellung der optisch aktiven Endgruppe des Capsorubins aus (3R)-3-Hydroxy-β-cyclocitral (vgl. Pure Appl. Chem. 51 (1979) 535-64) gelungen war, war der Weg für die Synthese von optisch aktivem Capsorubin frei (vgl. Helv. Chim. Acta 66, 7 (1983) Nr. 192, Seiten 1939 - 60).

Ein weiterer Zugang zu optisch aktivem Capsorubin gelang ausgehend von (+)-Campher (vgl. J. Mol. Struct. 226 (1992) Seiten 91-96) oder Isophoron (vgl. Quim. Nova 14 (1991) Seiten 22-25) als Quelle für den optisch aktiven Cyclopentylbaustein und Crocetindialdehyd. Nachteilig an allen bisher beschriebenen Synthesen des Capsorubins ist, daß die einzelnen Reaktionsschritte zu aufwendig und daher nicht für eine technische Durchführung geeignet sind.

Da sich die aus Helv. Chim. Acta 66 (1983) Seiten 1939 - 60 bekannte Aldolkondensation von (1R, 4S)-4-Hydroxy-1,2,2-trimethylcyclopentyl-methylketon mit Crocetindialdehyd jedoch auch in technischem Maßstab recht gut durchführen läßt, war es für die synthetische Herstellung von Capsorubin wichtig, einen leicht durchführbaren Weg zur Herstellung von (1R, 4S)-4-Hydroxy-1,2,2-trimethyl-cyclopentyl-methylketon der Formel I oder dessen O-geschützter Derivate zu entwickeln. 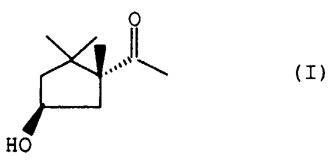 Es war daher die Aufgabe der Erfindung einen Weg zur Herstellung von (1R, 4S)-4-Hydroxy-1,2,2-trimethyl-cyclopentyl-methylketon und Derivaten ausgehend von einem leicht zugänglichen Ausgangsprodukt in möglichst wenigen und technisch leicht realisierbaren Reaktionsschritten zu entwickeln.

Gegenstand der Erfindung ist ein Verfahren zur Herstellung von 1,2,2-Trimethyl-cyclopentylmethylketon-derivaten der allgemeinen Formel I 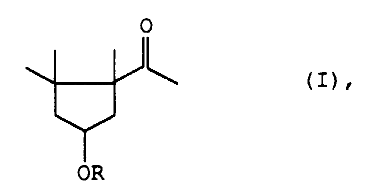 in der R für Wasserstoff oder einen Alkyl-, Aryl-, Arylmethyl-, Trialkylsilyl-, Triarylsilyl-, Alkylarylsilyl, Alkyloxyalkyl-, Tetrahydropyranyl-, Arylmethyloxycarbonyl-, Alkanoyl- oder Benzoyl-Rest, vorzugsweise für Wasserstoff oder einen tert.-Butyl-, Isopropyl-, Benzyl-, Alkylbenzyl-, 2-Naphthylmethyl-, tert.-Butyldimethylsilyl-, Benzyloxycarbonyl-, Tetrahydropyranyl-, Ethoxyethyl-, Acetyl-, Benzoyl- oder Methoxyisopropyl-Rest steht, das dadurch gekennzeichnet ist, daß man
A. ein 2,2,6-Trimethyl-cyclohexan-l-on der allgemeinen Formel II 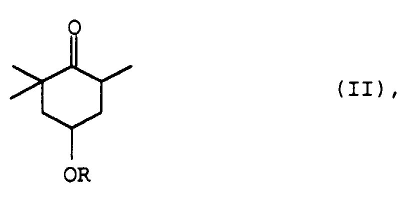 in der R für Wasserstoff oder einen der oben genannten Reste steht, mit einem Methylcarbanion der allgemeinen Formel III

   CH₃⁻M⁺ (III),

   in der M für Li, MgCl, MgBr oder MgJ steht, umsetzt
   und das erhaltene 1,4-Dihydroxy-1,2,2,6-tetramethyl-cyclohexan-derivat der allgemeinen Formel IV 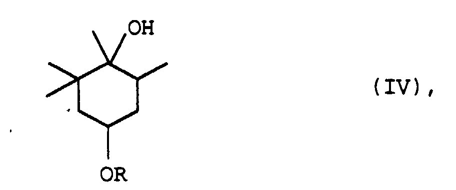 in der R für Wasserstoff, oder einen der oben genannte Reste steht, durch säurekatalysierte Eliminierung der tertiären Hydroxylgruppe in ein Gemisch der entsprechenden Verbindungen der Formeln V und VI 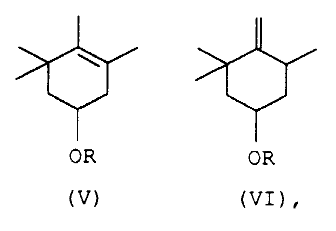 überführt,
   oder aber das 2,2,6-Trimethyl-cyclohexan-1-on der allgemeinen Formel II in einer Wittig-Reaktion mit einem Triphenylmethylenphosphoran der Formel VII

   (C₆H₅)₃P = CH₂ (VII)

   direkt in die Verbindung der allgemeinen Formel VI überführt,
B. die erhaltene Verbindung der allgemeinen Formel VI durch säurekatalysierte Doppelbindungsisomerisierung in die Verbindung der allgemeinen Formel V überführt,
C. die Doppelbindung in der Verbindung der allgemeinen Formel V 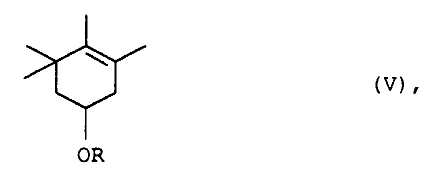 in der R die oben angegebene Bedeutung hat, in an sich bekannter Weise mit Peroxysäuren, deren Salzen oder Hydroperoxiden epoxidiert und
D. die erhaltenen 7-Oxa-bicyclo[4.1.0]heptan-derivate der allgemeinen Formel VIII 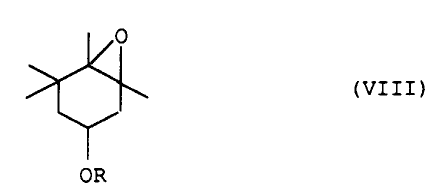 durch Umsetzen mit Lewis-Säuren und gewünschtenfalls durch Abspalten der Schutzgruppe am Sauerstoff in an sich bekannter Weise in die 1,2,2-Trimethyl-cyclopentylmethylketone der allgemeinen Formel I überführt.

Es war zwar aus "Carotenoid Chem. Biochem. Proc. Int. Symp. Carotenoids, 6th Meeting, Date 1981, Seiten 71-86, insbesondere 78-79, Edited by: Britton G.; Goodwen T.W., Pergamon Oxford" schon bekannt, daß man durch Epoxydieren optisch aktiver 1-Acetoxy-3,4,5,5-tetramethyl-3-cyclohexenderivate optisch aktive 3-Acetoxy-7-oxa-1,5,5,6-tetramethyl-bicyclo[4.1.0]heptane herstellen und diese durch Umsetzen mit Lewis-Säuren in optisch aktive 4-Hydroxy-1,2,2-trimethyl-cyclopentylmethylketone überführen kann, jedoch ist einerseits die hier beschriebene Herstellung der Ausgangsverbindung aus Hydroxycyclocitral technisch sehr aufwendig und andererseits kann durch die hier als Schutzgruppe verwendete Acetoxy-Gruppe keine ausreichende Stereokontrolle der Reaktion erzielt werden.

Für die Herstellung des als Capsorubinvorprodukt besonders begehrten (1R, 4S)-4-Hydroxy-1,2,2-trimethyl-cyclopentylmethylketons der allgemeinen Formel Ia 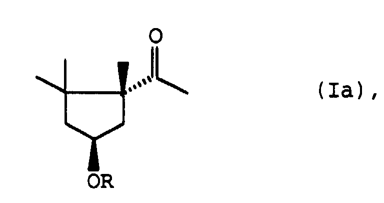 in der R für Wasserstoff steht, geht man im erfindungsgemäßen Verfahren so vor, daß man bei dem erfindungsgemäßen Verfahren im
Reaktionsschritt C. ein (1S)-3,4,5,5-Tetramethyl-3-cyclohexenderivat der allgemeinen Formel Va 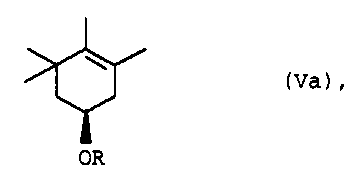 in der R für einen tert.-Butyl-, Isopropyl-, Benzyl-, Alkylbenzyl-, 2-Naphthylmethyl- oder tert.-Butyldimethylsilylrest steht, stereoselektiv epoxidiert und im Reaktionsschritt
D. das so erhaltene (1R, 3S, 6S)-7-Oxa-bicyclo[4.1.0]heptan-derivat der allgemeinen Formel VIIIa 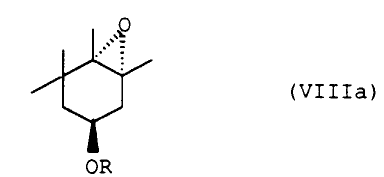 mit einer Lewis-Säure umsetzt und anschließend die Schutzgruppe in an sich bekannter Weise abspaltet.
   Mit Vorteil geht man erfindungsgemäß zur Herstellung eines ggf. mit einer Schutzgruppe versehenen (1R, 4S)-4-Hydroxy-1,2,2-trimethyl-cyclopentylmethylketons der allgemeinen Formel Ia 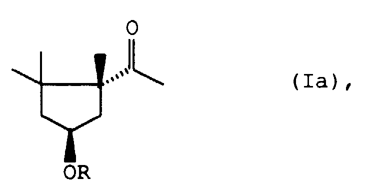 in der R für Wasserstoff, einen tert.-Butyl-, Isopropyl-, Benzyl-, Alkylbenzyl-, 2-Naphthylmethyl- oder tert.-Butyldimethylsilylrest steht, so vor, daß man im Reaktionsschritt
A. ein (4R, 6R)-4-Hydroxy-2,2,6-trimethyl-cyclohexan-1-on der allgemeinen Formel IIa 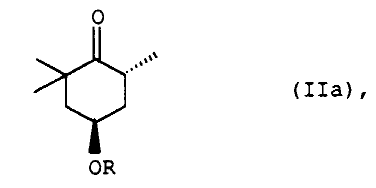 in der R für Wasserstoff steht, einsetzt, dieses gemäß den oben beschriebenen Reaktionsschritten A. und B. in das (1S)-3,4,5,5-Tetramethyl-3-cyclohexen-1-ol der allgemeinen Formel Va 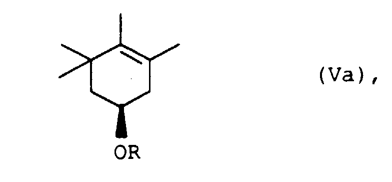 in der R für Wasserstoff steht, überführt und anschließend in an sich bekannter Weise die Hydroxylgruppe in Va mit einer voluminösen, nicht koordinierenden und komplexierenden Schutzgruppe versieht,
C. das so erhaltene (1S)-3,4,5,5-Tetramethyl-3-cyclohexen-1-ol-derivat der allgemeinen Formel Va 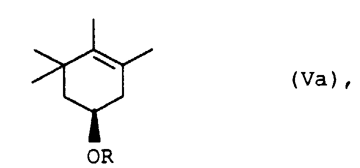 in der R vorzugsweise für einen tert.-Butyl-, Isopropyl-, Benzyl-, Alkylbenzyl-, 2-Naphthylmethyl-, tert.-Butyldimethylsilylrest steht, stereoselektiv epoxidiert und
D. das so erhaltene (1R,3S,6S)-7-Oxa-bicyclo[4.1.0]heptan-derivat der allgemeinen Formel VIIIa 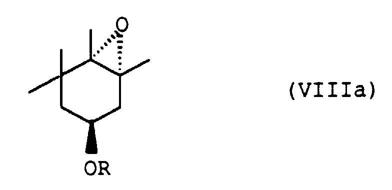 in der R die unter C. für Verbindung Va angegebene Bedeutung hat mit einer Lewis-Säure umsetzt und gewünschtenfalls die Schutzgruppe in an sich bekannter Weise entfernt.
   Zur Herstellung von einem (1S,4S)-1,2,2-Trimethyl-cyclopentylmethylketon der allgemeinen Formel Ib 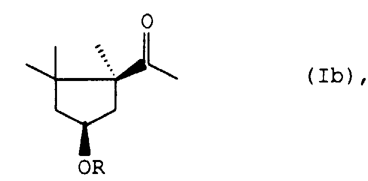 in der R für Wasserstoff oder einen Benzyloxycarbonyl-, Tetrahydropyranyl-, Ethoxyethyl-, Acetyl- oder Methoxyisopropyl-Rest steht, geht man erfindungsgemäß im allgemeinen so vor, daß man im Reaktionsschritt C ein (1S)-3,4,5,5-Tetramethyl-3-cyclohexen-1-ol-derivat der allgemeinen Formel Va 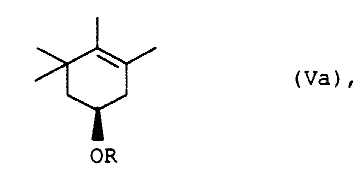 in der R für Wasserstoff oder einen der oben für das Keton Ib genannten Reste steht, diastereoselektiv epoxidiert,
D. das erhaltene (1S,3S,6R)-7-Oxa-1,5,5,6-tetramethyl-bicyclo[4.1.0]heptan der allgemeinen Formel VIIIb 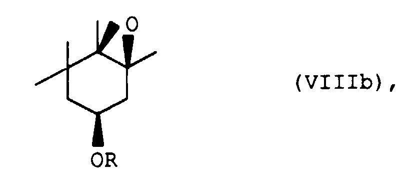 mit einer Lewis-Säure umsetzt und gewünschtenfalls die Schutzgruppe in an sich bekannter Weise entfernt.
   Mit Vorteil geht man erfindungsgemäß zur Herstellung von einem (1S,4S)-1,2,2-Trimethyl-cyclopentylmethylketon der allgemeinen Formel Ib 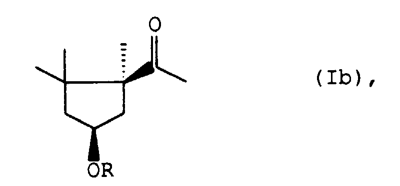 in der R für Wasserstoff steht, so vor, daß man ein (4R,6R)-2,2,6-Trimethyl-cyclohexan-1-on-derivat der allgemeinen Formel IIb 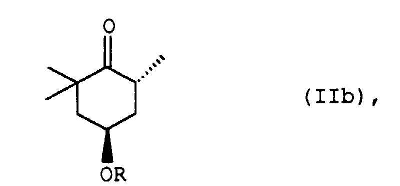 in der R für H oder einen Benzyloxycarbonyl-, Tetrahydropyranyl-, Ethoxyethyl-, Acetyl- oder Methoxyisopropyl-Rest steht, einsetzt, dieses gemäß den oben beschriebenen Reaktionsschritten A. und B. in ein (1S)-3,4,5,5-Tetramethyl-3-cyclohexen-derivat der allgemeinen Formel Va 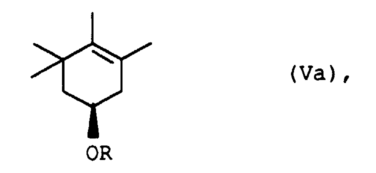 in der R für Wasserstoff oder einen der oben für das Cyclohexanon IIb genannten Reste steht, überführt,
C. dieses stereoselektiv epoxydiert und
D. das so erhaltene (1S,3S,6R)-7-Oxa-1,5,5,6-tetramethyl-bicyclo[4.1.0]heptan-derivat der allgemeinen Formel VIIIb 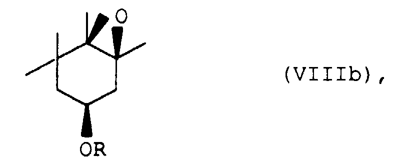 mit einer Lewis-Säure umsetzt und die Schutzgruppe in an sich bekannter Weise entfernt.

Von den Zwischenprodukten dieses vorteilhaften Verfahrens der Erfindung sind die 1,4-Dihydroxy-1,2,2,6-tetramethyl-cyclohexan-derivate der allgemeinen Formel IV 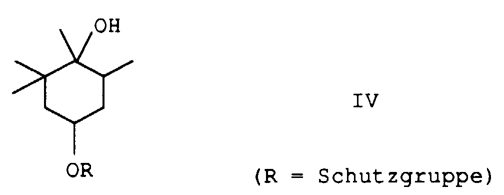 sowie deren (1S)- und (1R)-Isomeren;
die 3,4,5,5-Tetramethyl-3-cyclohexen-derivate der allgemeinen Formel V 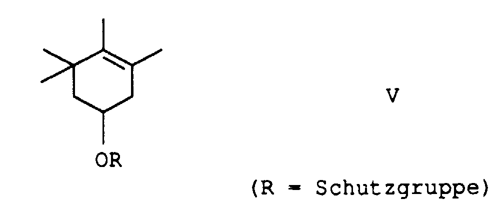 sowie deren (1S)- und (1R)-Isomeren;
die 3,5,5-Trimethyl-4-methylen-cyclohexan-derivate der allgemeinen Formel VI 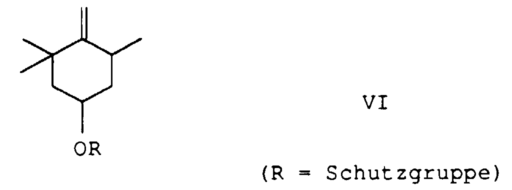 sowie deren (1S)- und (1R)-Isomeren;
und die 7-Oxa-1,5,5,6-tetramethyl-bicyclo[4.1.0]heptan-derivate der allgemeinen Formel VIII 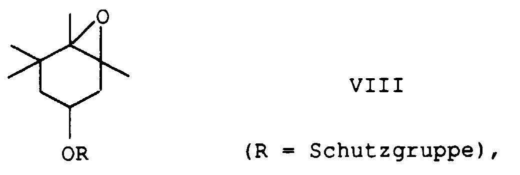 sowie deren (1R, 3S, 6S)-, (1R,3R,6S)- und (1S,3S,6R)-Isomeren neu und werden soweit wie möglich beansprucht.

Die als Ausgangsprodukte für das erfindungsgemäße Verfahren benötigten 4-Hydroxy-2,2,6-Trimethyl-cyclohexan-1-one der allgemeinen Formel II 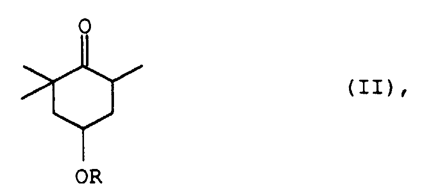 in der R für Wasserstoff steht, können ausgehend von dem technisch gut zugänglichen Oxoisophoron durch fermentative Reduktion und anschließende chemische Reduktion des erhaltenen optisch aktiven gesättigten Ketons erhalten werden, wobei bei geeigneter Wahl der Bedingungen überwiegend das für die Synthese von Capsorubin benötigte (4R, 6R)-Isomere gebildet wird (vgl. Helv. Chim. Acta 59 (1976) Seiten 1832-49, besonders 1839).

Die Einführung von Schutzgruppen in die Hydroxygruppe des Cyclohexanons kann in an sich bekannter Weise erfolgen.

Für die Überführung der 2,2,6-Trimethyl-cyclohexan-1-on-derivate der allgemeinen Formel II in die 1,4-Dihydroxy-cyclohexan-derivate der allgemeinen Formel IV verwendet man als Methylcarbanionen mit Vorteil Methylgrignardverbindungen, wie CH₃MgCl, CH₃MgBr und CH₃MgJ, oder aber Methyl-Lithium. Bezüglich näherer Einzelheiten über die Durchführung der Grignard-Reaktion bzw. der Umsetzung mit Methyllithium verweisen wir beispielsweise auf "Organikum, Organisch-chemisches Grundpraktikum", 16. Auflage, VEB Deutscher Verlag der Wissenschaften, Berlin 1986, Seiten 499-502. Die Umsetzung wird im allgemeinen unter einem Schutzgas, wie N₂, durchgeführt.

Für die säurekatalysierte Eliminierung der tertiären Hydroxylgruppe aus dem 1,4-Dihydroxy-1,2,2,6-tetramethyl-cyclohexan der allgemeinen Formel IV haben sich folgende Reaktionsbedingungen besonders bewährt: In einem inerten organischen Lösungsmittel, das mit Wasser ein Azeotrop bildet, werden die 1,4-Dihydroxy-cyclohexanderivate der allgemeinen Formel IV unter Zusatz von Spuren einer Säure, wie H₂SO₄, H₃PO₄ oder p-Toluolsulfonsäure, kurzzeitig unter Rückfluß zum Sieden erhitzt, wodurch eine selektive Eliminierung der tertiären und nicht der sekundären Hydroxygruppe gelingt. Gleichzeitig besteht unter diesen Bedeutungen die Möglichkeit zu einer quantitativen Isomerisierung der exo-Methylengruppe in Verbindungen der allgemeinen Formel VI in die interne Doppelbindung von Verbindungen der allgemeinen Formel V.

Auch die Umsetzung der 2,2,6-Trimethyl-cyclohexan-1-on-derivate der allgemeinen Formel II mit Triphenylmethylenphosphoranen der Formel VII in die Methylencyclohexan-Verbindungen der allgemeinen Formel VI erfolgt in an sich bekannter Weise. Bezüglich näherer Einzelheiten über die Durchführung von Wittig-Reaktionen mit cyclischen Ketonen verweisen wir beispielsweise auf "Organikum, Organisch-chemisches Grundpraktikum" 16. Auflage, VEB Deutscher Verlag der Wissenschaften, Berlin 1986, Seiten 465-66.

Die säurekatalysierte Doppelbindungsisomerisierung von der Methylencyclohexan-Verbindungen der allgemeinen Formel VI in die Tetramethylcyclohexen-1-derivate der allgemeinen Formel V 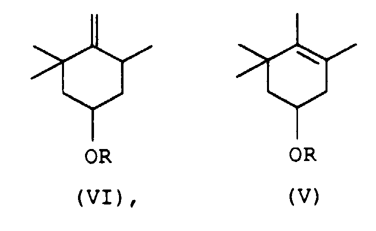 erfolgt im allgemeinen in inerten organischen Lösungsmitteln, wie aliphatischen oder aromatischen Kohlenwasserstoffen oder Halogenkohlenwasserstoffen unter Zusatz katalytischer Mengen einer Säure, wie H₂SO₄, H₃PO₄ oder p-Toluolsulfonsäure, bei Temperaturen von 60 bis 150°C, vorzugsweise 80 bis 140°C, insbesondere 90-120°C.

Für die Isomerisierungsreaktion setzt man entweder das bei der Umsetzung mit Methylcarbanionen und nachfolgende Wassereliminierung erhaltene Gemisch aus den Verbindungen der Formel V und VI ein oder aber das bei der Umsetzung mit dem Triphenylmethylenphosphoran erhaltene Methylencyclohexan der Formel VI.

Für die nachfolgende Epoxidierung der Doppelbindung in den Verbindungen der allgemeinen Formel V haben sich folgende Reaktionsbedingungen besonders bewährt:

Als Epoxidierungsmittel verwendet man im allgemeinen Peroxysäuren, wie Peroxyameisensäure, Peroxyessigsäure, Peroxybenzoesäure, Peroxytrifluoressigsäure, Monoperoxyphthalsäure, Perwolframsäure, Permolybdänsäure oder 3-Chlor-peroxybenzoesäure, Salze von Peroxysäuren, wie Magnesiumperoxyphthalat oder Pervanadate, oder Hydroperoxide, wie tert.-Butylhydroperoxid/VO(CH₃-CO-CH₂-CO-)₃; tert.-Butylhydroperoxid/MnO₂ oder VO(CH₃-CO-CH₂-CO-)₃/H₂O₂. Das Epoxydierungsmittel verwendet man im allgemeinen in Mengen von 1 bis 1,8, vorzugsweise 1 bis 1,5, insbesondere 1 bis 1,2 Mol pro Mol der Verbindung der allgemeinen Formel V, Va oder Vb.

Als Lösungsmittel für diese Epoxidierung sind aliphatische oder aromatische Kohlenwasserstoffe oder Halogenkohlenwasserstoffe besonders geeignet.

Geeignete Reaktionstemperaturen sind Temperaturen von -30 bis +40°C, vorzugsweise -10 bis +30°C, insbesondere -5 bis +25°C.

Die Epoxidierung wird mit Vorteil unter Lichtausschluß durchgeführt.

Die Epoxydierung von (1S)-3,4,5,5-Tetramethyl-3-cyclohexen-1-ol der Formel Va 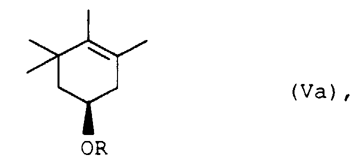 in der R für H steht, ermöglicht unter Ausnutzung der asymmetrischen Induktion, eine diastereoselektive Synthese von (1S,3S,6R)-7-Oxa-1,5,5,6-tetramethyl-bicyclo[4.1.0]heptan der allgemeinen Formel VIIIb 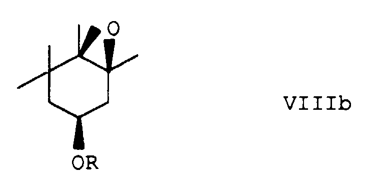 in der R für H steht.
Die Einführung von Schutzgruppen R mit starker Donorfunktion, die zur Ausbildung von Wasserstoffbrückenbindungen führen bzw. zur Komplexierung von Epoxidierungskatalysatoren dienen, ermöglicht eine Erhöhung der Stereoselektivität für diese Art der Reaktion.
Als Schutzgruppen mit starker Donorfunktion seien beispielsweise genannt: Arylmethyloxycarbonylgruppen, wie die Benzyloxycarbonylgruppe, Alkyloxyalkylgruppen, wie die Ethoxyethylgruppe und die Methoxyisopropylgruppe, Alkanoylgruppen, wie die Acetylgruppe, Arylalkanoylgruppen, wie die Benzoylgruppe oder eine Tetrahydropyranoylgruppe.

Führt man dagegen an der Hydroxygruppe von (1S)-3,4,5,5-Tetramethyl-3-cyclohexen-1-ol der Formel Va Schutzgruppen mit stark voluminösem Charakter ein, so wird die Diastereoselektivität der Epoxidierungsreaktion invertiert.

Als Schutzgruppen mit stark voluminösem, nicht koordinierendem und nicht komplexierendem Charakter seien beispielsweise folgende Gruppen genannt: voluminöse Alkylgruppen, wie die tert.-Butylgruppe oder die Isopropylgruppe, Arylgruppen, wie die Phenyl-, eine Alkylphenyl-, eine Naphthyl- oder eine Alkylnaphthylgruppe, oder aber Arylmethylgruppen, wie die Benzylgruppe oder 2-Naphthyl-methylgruppe, eine Alkylarylmethylgruppe, wie die tert.-Butyl-benzylgruppe, eine Trialkylsilylgruppe, wie die tert.-Butyldimethylsilylgruppe, eine Triarylsilylgruppe, wie die Triphenylsilylgruppe oder eine Alkylarylsilylgruppe, wie die Dimethylphenylsilylgruppe.

Werden die so stereochemisch unterschiedlich aufgebauten Epoxide mit Lewis-Säuren umgesetzt, so läßt sich in kurzer Zeit eine Ringverengung unter Ausbildung der entsprechenden Cyclopentylmethylketone erreichen. Diese Ringverengungen verlaufen mit hoher Selektivität stark stereokontrolliert. Während das Epoxid mit syn-Orientierung der Hydroxygruppierung bei der Ringverengung (1S,4S)-1,2,2-Trimethyl-cyclopentylmethylketone der Formel Ib ergibt, liefert die Ringverengung der Epoxidverbindung mit anti-Orientierung der Hydroxyfunktion das für die Herstellung des begehrten Capsorubins benötigte (1R,4S)-4-Hydroxy-1,2,2-trimethylcyclopentylmethylketon bzw. dessen Derivate der Formel Ia.

Verwendet man dagegen als Ausgangsverbindung für das erfindungsgemäße Verfahren (4S,6R)-4-Hydroxy-2,2,6-trimethyl-cyclohexan-1-on-derivate der allgemeinen Formel IIc 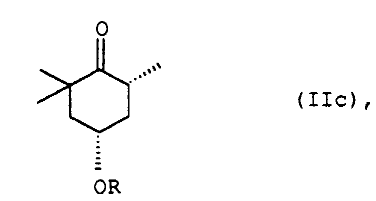 in der R für Wasserstoff oder einen Benzyloxycarbonyl-, Tetrahydropyranyl-, Ethoxyethyl-, Acetyl-, Benzoyl-, Alkylbenzoyloder Methoxiisopropenylrest steht, so kommt man durch die erfindungsgemäßen Reaktionsschritte A. und B. zu einem (1R)-3,4,5,5-Tetramethyl-3-cyclohexen der allgemeinen Formel Vc, aus dem man durch Epoxidieren stereoselektiv ein (1R,3R,6S)-7-Oxa-1,5,5,6-tetramethyl-bicyclo[4.1.0]heptan-derivat der allgemeinen Formel VIIIc und daraus durch Umsetzen mit Lewis-Säuren unter Ringverengung und Abspaltung der Schutzgruppen ein (1R,4R)-1,2,2-Trimethyl-cyclopentylmethylketon der Formel Ic herstellen kann. 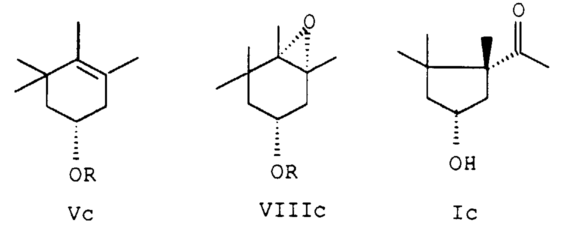 Eine Umwandlung von [1R,4R]-4-Hydroxy-1,2,2-trimethyl-cyclopentylmethylketon in [1R,4S]-4-Hydroxy-1,2,2-trimethyl-cyclopentylmethylketon ist nach vorhergehender Ketalisierung durch Oxidation, enantioseleketive Reduktion und abschließende Ketalspaltung ebenfalls möglich.

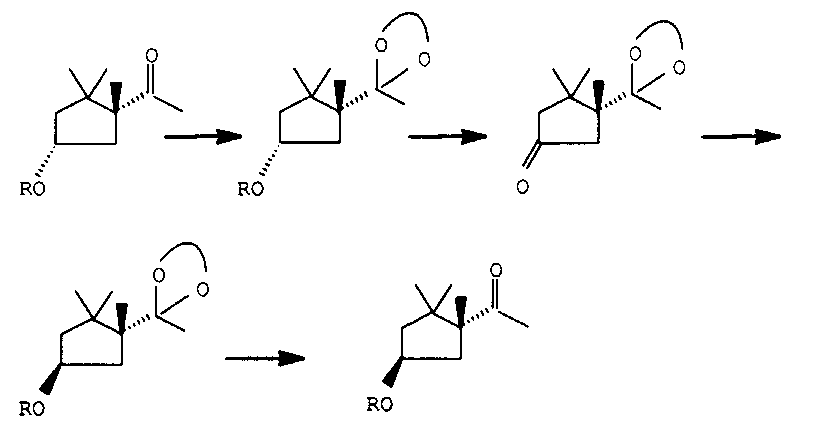

Diese Sequenz ist jedoch infolge der deutlich höheren Stufenzahl für die Herstellung von Capsorubin weniger von Bedeutung.

Als Lewis-Säuren kann man erfindungsgemäß beispielsweise einsetzen: MgCl₂, MgBr₂, MgJ₂, CaCl₂, CaBr₂, FeCl₃, AlCl₃, TiCl₄, SnCl₄, BF₃·(C₂H₅)₂O, SbCl₃ oder ZnCl₂.

Mit Vorteil verwendet man FeCl₃, AlCl₃, TiCl₄; BF₃·(C₂H₅)₂O, oder SnCl₄, insbesondere FeCl₃, BF₃·(C₂H₅)₂O oder AlCl₃. Die Lewis-Säuren verwendet man in Mengen von etwa 0,01 bis 1 Äquivalent pro Äquivalent der Epoxiverbindung.

Es war sehr überraschend, daß die Ringverengung der Epoxide der allgemeinen Formel VIIIa-c nahezu stereoselektiv verläuft und darüber hinaus bei der Ringverengung im wesentlichen nur ein Reaktionsprodukt, aktionsprodukt, nämlich Cyclopentylmethylketone der allgemeinen Formeln I bzw. Ia, Ib und Ic gebildet werden.

Mit Hilfe des erfindungsgemäßen Verfahrens kann das für die technische Herstellung von Capsorubin benötigte (1R,4S)-4-Hydroxy-1,2,2-trimethyl-cyclopentylmethylketon der Formel Ia mit R=H sowie Derivate oder Stereoisomere dieser Verbindung ausgehend von einem technisch leicht zugänglichen Ausgangsstoff auf technisch relativ einfache Weise und mit recht guten Ausbeuten über neue Zwischenprodukte hergestellt werden.

### Beispiel 1

### Synthese von (1S,4S)-4-Hydroxy-1,2,2-trimethyl-cyclopentylmethylketon (Ib)

### A. Herstellung von (4R)-1,4-Dihydroxy-1,2,2,6-tetramethyl-cyclohexan

In 9 ml einer 3 m Lösung von Methylmagnesiumbromid (26,6 mmol) in Diethylether(Ether) ließ man unter Aufrecherhalten von Raumtemperatur (RT) durch Außenkühlung unter Stickstoff als Schutzgas innerhalb von 15 Minuten (min) eine Lösung von 1,11 g (7,2 mmol) (4R,6R)-4-Hydroxy-2,6,6-trimethyl-cyclohexan-1-on in 50 ml absolutem Tetrahydrofuran (THF) hineintropfen. Man erhielt dabei eine trübe, gelblich gefärbte Lösung, die noch 12 Stunden (h) bei RT und N2 nachgerührt wurde. Anschließend wurde das Reaktionsgemisch mit 10 ml einer 5 gew.-%igen wäßrigen Schwefelsäure versetzt und 2 mal mit 50 ml Ether extrahiert. Durch Abdestillieren des Lösungsmittels wurden 1,05 g eines Rohproduktes erhalten. Es handelte sich um ein Diastereomerengemisch, bezogen sowohl auf das Zentrum 1 als auch das Zentrum 6.

### B. Herstellung von (1S)-3,4,5,5-Tetramethyl-3-cyclohexen-1-ol

4 g (23,2 mmol) eines gemäß Beispiel 1A erhaltenen Diastereomerengemisches wurden in 50 ml Toluol aufgenommen, die Lösung mit einer Spatelspitze (etwa 4 mg) p-Toluolsulfonsäure (p-TsOH) versetzt und 45 min unter Rückfluß zum Sieden erhitzt. Anschließend wurde das Reaktionsgemisch in 50 ml Wasser gegeben und 2 mal mit 50 ml Ether extrahiert. Die erhaltene organische Phase wurde mit 50 ml einer gesättigten wäßrigen NaHCO₃-Lösung gewaschen, über MgSO₄ getrocknet und unter vermindertem Druck vom Lösungsmittel befreit. Das erhaltene Rohprodukt wurde an 150 g Kieselgel mit einem Hexan/Essigsäureethylester (ESE)-Gemisch im Verhältnis (4:1) chromatographiert. Man erhielt das gewünschte Produkt in Form von 2,5 g hellgelber Kristalle in einer Reinheit von 96,4 % (GC-Analyse). Das entspricht einer Selektivität von 71 % der Theorie.

### C. Herstellung von (1S,3S,6R)-3-Hydroxy-7-Oxa-1,5,5,6-tetramethyl-bicyclo[4.1.0]heptan

2,5 g (16,2 mmol) des gemäß Beispiel 1B erhaltenen (1S)-3,4,5,5-Tetramethyl-3-cyclohexen-1-ols wurden unter N₂-Schutz in 50 ml Methylenchlorid gelöst, die Lösung auf 0°C abgekühlt und danach unter Lichtausschluß mit 5,1 g einer 55 gew.-%igen Lösung von 3-Chlor-peroxybenzoesäure in Methylenchlorid (MCPBA; entsprechend 16,2 mmol) versetzt und das Reaktionsgemisch noch 1 h bei RT gerührt. Anschließend wurde das Reaktionsgemisch in 50 ml Eiswasser eingetragen, mit 50 ml Methylenchlorid versetzt. Nach intensivem Vermischen wurde die organische Phase abgetrennt, mit 100 ml Wasser, dann mit 50 ml einer 5%igen Na₂CO₃-Lösung und dann mit 50 ml Wasser gewaschen, anschließend getrocknet und vom Lösungsmittel befreit. Man erhielt 2,56 g (entsprechend 93 % der Theorie) der gewünschten Epoxydverbindung. Durch Chromatographie an 75 g Kieselgel mit Hexan/ESE (4:1) erhielt man 2,05 g der gewünschten Verbindung. Die Ausbeute an reinem Produkt betrug 75 %, die Aufklärung der relativen Konfiguration gelang durch Anwendung von 2 D-NMR-Experimenten.

### D. Herstellung von (1S, 4S)-4-Hydroxy-1,2,2-trimethyl-cyclopentylmethylketon

1 g (5,87 mmol) des gemäß Beispiel 1C erhaltenen (1S, 3S, 6R)-3-Hydroxy-7-oxa-1,5,5,6-tetramethyl-bicyclo[4.1.0]heptans wurden in 25 ml absolutem (über Alox B filtriertem) Methylenchlorid gelöst und die Lösung bei RT 2mal mit 0,36 ml (5,88 mmol) BF₃x(C₂H₅)₂O versetzt und dann 2 h bei RT gerührt. Anschließend wurde das Reaktionsgemisch mit 25 ml Methylenchlorid verdünnt, mit 25 ml Wasser gewaschen und dann getrocknet. Durch Entfernen des Lösungsmittels unter vermindertem Druck wurden 0,92 g eines rotbraunen Rohproduktes erhalten. Chromatographie an 50 g Kieselgel mit Hexan/ESE (5:1) ergab 321 mg des oben genannten Cyclopentylmethylketons. Der Vergleich der Spektren der isolierten Verbindung mit Literaturdaten ergab eine sehr gute Übereinstimmung. Die Bestimmung der relativen Konfiguration des neu im System entstandenen Stereozentrums erfolgte über CH-Correlations- und ROESY-Spektroskopie (NMR-spektroskopische Methode zur zweifelsfreien Zuordnung von H-Resonanzen und deren räumlichem Verhältnis zueinander).

### Beispiel 2

### Synthese von (1R, 4S)-4-Hydroxy-1,2,2-trimethyl-cyclopentylmethylketon (Ia)

### A. Herstellung von (1R)-3,3,5-Trimethyl-4-methylen-cyclohexan-1-ol

Eine Lösung von 2,5 g (6,86 mmol) Methyltriphenylphosphoniumbromid in 10 ml Dimethylsulfoxid (DMSO) wurde unter N₂-Schutzgas mit 0,79 g (6,86 mmol) Kalium-tert-butylat (KOtBu) versetzt und anschließend 30 min bei RT gerührt. Zu diesem Gemisch wurden 0,36 g (2,29 mmol) (4R, 6R)-4-Hydroxy-2,2,6-trimethyl-cyclohexan-1-on addiert und das Reaktionsgemisch noch 5 min bei RT nachgerührt, wobei ein weißer Niederschlag ausfiel. Anschließend wurde das Reaktionsgemisch für 15 min bei 60°C gerührt, dann auf RT abgekühlt, in 150 ml Eiswasser gegossen und 2mal mit 50 ml Methylenchlorid extrahiert. Nach Waschen der abgetrennten organischen Phase mit 50 ml einer 5%igen wäßrigen Na₂CO₃-Lösung und 50 ml Wasser sowie Trocknen über MgSO₄ wurde das Lösungsmittel unter vermindertem Druck entfernt.

### B. Herstellung von (1S)-3,4,5,5-Tetramethyl-3-cyclohexen-1-ol

4 g eines gemäß Beispiel 2A erhaltenen (1R)-3,3,5-Trimethyl-4-methylen-cyclohexan-1-ols wurden in 50 ml Toluol gelöst, mit ca. 6 mg p-Toluolsulfonsäure versetzt und für 45 min unter Rückfluß zum Sieden erhitzt. Anschließend wurde das Reaktionsgemisch in 50 ml Wasser gegeben und 2mal mit 50 ml Ether extrahiert. Die abgetrennte organische Phase wurde mit 50 ml einer gesättigten NaHCO₃-Lösung gewaschen, über MgSO₄ getrocknet und unter vermindertem Druck vom Lösungsmittel befreit. Der Rückstand wurde an 150 g Kieselgel mit Hexan/ESE (4:1) chromatographiert. Man erhielt 3,7 g des oben genannten Produkts in Form hellgelber Kristalle, das nach GC 97 % rein war.

### C. Herstellung von (1S)-1-tert.-Butyldimethylsilyloxy-3,4,5,5-tetramethyl-3-cyclohexen

0,8 (5,2 mmol) des Beispiel 2B erhaltenen (1S)-3,4,5,5-Tetramethyl-3-cyclohexen-1-ol wurden in 5 ml absolutem Methylenchlorid gelöst, die Lösung mit 0,5 ml Pyridin versetzt und auf 0°C abgekühlt. Anschließend wurde mit 1,54 g (5,7 mmol) Trifluormethansulfonsäure-tert.-butyldimethylsilylester versetzt, das Reaktionsgemisch noch 15 min bei 0°C gerührt und dann in 10 ml Wasser gegossen. Nach Extraktion mit 10 ml Methylenchlorid wurde die organische Phase mit 10 ml einer 0,1 m H₂SO₄, 10 ml einer gesättigten NaHCO₃-Lösung und 10 ml Wasser gewaschen, über MgSO₄ getrocknet und eingeengt. Man erhielt 1,3 g (92 % der Theorie) des oben genannten Produktes.

### D. Herstellung von (1R, 3S, 6S)-3-(tert.-Butyldimethylsilyloxy) -7-oxa-1,5,5,6-tetramethyl-bicyclo[4.1.0]heptan

1,25 g (4,7 mmol) des gemäß Beispiel 1C erhaltenen (1S)-1-tert.-Butyldimethylsilyloxy-3,4,5,5-tetramethyl-3-cyclohexens wurden in 25 ml absolutem Methylenchlorid gelöst, die Lösung auf 0°C abgekühlt, dann mit 1,5 g 3-Chlor-peroxybenzoesäure in Methylenchlorid (MC PBA; entsprechend 4,7 mmol) versetzt, 1 h bei 0°C gerührt und dann in 25 ml Eiswasser eingetragen. Anschließend wurden noch 25 ml Methylenchlorid zugefügt, intensiv vermischt, die abgetrennte organische Phase mit 25 ml einer 5%igen Na₂SO₃-Lösung und 25 ml Wasser gewaschen, getrocknet und vom Lösungsmittel befreit. Man erhielt 1,18 g (88 % d. Theorie) farbloser Kristalle. Der Diastereomerenüberschuß liegt gemäß NMR-Analyse bei 20 %.

### E. Herstellung von (1R,4S)-4-Hydroxy-1,2,2-trimethyl-cyclopentylmethylketon

1,1 g (3,87 mmol) des gemäß Beispiel 2 D erhaltenen (1R,3S,6S)-3-(tert.-Butyldimethylsilyloxy)-7-oxa-1,5,5,6-tetramethyl-bicyclo[4.1.0]heptans wurden in 25 ml Methylenchlorid gelöst, die Lösung bei RT mit 0,24 ml (1,93 mmol) BF₃(C₂H₅)₂O versetzt, das Reaktionsgemisch 1 h bei RT gerührt, dann mit 25 ml Wasser versetzt und die abgetrennte organische Phase mit Wasser gewaschen und eingeengt. Zur quantitativen Entfernung der Schutzgruppe wurden dem Rückstand 3,7 ml (6,1 mmol) einer 1,1 m Tetrabutylammoniumfluoridlösung zugesetzt. Es wurde erneut mit Wasser gewaschen und die organische Phase abgetrennt. Chromatographie an Kieselgel mit einem Hexan/ESE (5:1)-Gemisch ergab 0,9 g eines Produktes mit einem Diastereomerenüberschuß von 20 %.

### Beispiel 3

### Synthese von (1R, 4S)-4-Hydroxy-1,2,2-trimethyl-cyclopentylmethylketon (Ia)

### A. Herstellung von (1S)-1-Benzyloxy-3,4,5,5-tetramethyl-3-cyclohexen

2 g (13 mmol) eines gemäß Beispiel 1A und 1B hergestellten (1S)-3,4,5,5-Tetramethyl-3-cyclohexen-1-ols wurden in 10 ml Dimethoxyethan (DME) gelöst und zur Alkoholatbildung bei RT zu einer Suspension von 965 mg (38.9 mmol) NaH in 10 ml DME getropft. Nach abgeschlossener Gasentwicklung wurden 14 mmol Benzylbromid zugetropft. Nach 12 h Rühren bei RT lag vollständiger Umsatz vor. Der Ansatz wurde dann mit 50 ml Wasser versetzt und 2mal mit 50 ml Ether extrahiert. Chromatographie des Rückstands mit Hexan/ESE(4:1) ergab 3 g (86 %) des obengenannten Produktes.

### B. Herstellung von (1R, 3S, 6S)-3-Benzyloxy-7-oxa-1,5,5,6-tetramethyl-bicyclo[4.1.0]heptan

1,9 g (7.77 mmol) des gemäß Beispiel 3A hergestellten (1S)-1-Benzyloxy-3,4,5,5-tetramethyl-3-cyclohexens wurden in 40 ml Methylenchlorid gelöst und auf 0°C abgekühlt. Man setzte 2.44 g 3-Chlor-peroxybenzoesäure in Methylenchlorid (entsprechend 7,77 mmol)zu und rührte 1 h bei 0°C nach; anschließend wurde auf 150 ml Eiswasser gegeben und 2mal mit 50 ml Methylenchlorid extrahiert. Die organischen Phasen wurden dann mit 50 ml einer 5%igen Natriumsulfit-Lösung und 50 ml Wasser gewaschen, getrocknet und vom Lösungsmittel befreit. Man erhielt 2 g (96 %) des Produkts. Der Diastereomerenüberschuß beträgt 65 %.

### C. Herstellung von (1R,4S)-4-Hydroxy-1,2,2-trimethyl-cyclopentylmethylketon

1,64 g (6,5 mmol) des gemäß Beispiel 3B hergestellten (1R,3S,6S)-3-Benzyloxy-7-oxa-1,5,5,6-tetramethylbicyclo(4.1.0]heptans wurden in 25 ml Methylenchlorid gelöst und bei RT mit 0,4 ml Bortrifluorid-Etherat versetzt. Man ließ 1 h bei RT nachrühren. Aufarbeitung erfolgte analog Beispiel 2E. Man erhielt nach Chromatographie 0,98 g Produkt. Zur Abspaltung der Schutzgruppe wurde der Rückstand in 50 ml Ethanol aufgenommen, mit 150 mg 10 % Pd/C versetzt und unter Normaldruck bei RT hydriert. Nach 45 min war die Hydrierung beendet. Der Katalysator wurde abgetrennt und das Lösungsmittel unter vermindertem Druck entfernt. Man erhielt 0,96 g des oben genannten Produkts mit einer Diastereoselektivität von 65 %.

### Beispiel 4

### Synthese von (1R,4S)-4-Hydroxy-l,2,2-trimethyl-cyclopentylmethylketon (Ia)

### A. Herstellung von (1S)-1-(2-Naphthylmethyl-oxy)-3,4,5,5-tetramethyl-3-cyclohexen

2 g (13 mmol) eines gemäß Beispiel 2A und 2B hergestellten (1S)-3,4,5,5-Tetramethyl-3-cyclohexen-1-ols wurden in 10 ml DME gelöst und bei RT zu einer Suspension von 965 mg (38,9 mmol) NaH in 10 ml DME getropft. Nach abgeschlossener Gasentwicklung wurden 14 mmol 2-Brom-methylnaphthalin zugetropft. Nach 12 h Rühren bei RT war der Umsatz vollständig. Das Reaktionsgemisch wurde mit 50 ml Wasser versetzt und dann 2mal mit 50 ml Ether extrahiert. Durch Chromatographie des Rückstands analog Beispiel 3A wurden 3,7 g (97 %) des oben genannten Produkts erhalten.

### B. Herstellung von (1R,3S,6S)-3-(2-Naphthylmethyl-oxy)-7-oxa-1,5,5,6-tetramethylbicyclo[4.1.0]heptan

4 g (13,6 mmol) eines gemäß Beispiel 4A hergestellten (1S)-1-(2-Naphthylmethyl-oxy)-3,4,5,5-tetramethyl-3-cyclohexens wurden analog Beispiel 2D mit 4,3 g (13,6 mmol) 55 %iger MCPBA umgesetzt.
Man erhielt 4,1 g des oben genannten Produkts (entsprechend 97 % der Theorie) mit einer Diastereoselektivität von 68 %.

### C. Herstellung von (1R,4S)-4-Hydroxy-1,2,2-trimethyl-cyclopentylmethylketon

4 g (1,3 mmol) (1R,3S,6S)-3-(2-Naphthylmethyloxy)-7-oxa1,5,5,6-tetramethylbicyclo[4.1.0]heptan wurden analog Beispiel 3C in Methylenchlorid mit Bortrifluoridetherat umgesetzt. Nach der Hydrierung wurde der Katalysator abgetrennt, das Lösungsmittel entfernt. Man erhielt das oben genannte Produkt mit 78 % Diastereoselektivität. Die Trennung der Diastereomeren erfolgte durch einfache Destillation.

### Beispiel 5

### Synthese von (1R,4S)-4-Hydroxy-1,2,2-trimethyl-cyclopentylmethylketon (Ia)

### A. Herstellung von (1S)-1-(4-tert.-Butylbenzyl-oxy)-3,4,5,5-tetramethyl-3-cyclohexen

Durch Umsetzen von (1S)-3,4,5,5-Tetramethyl-3-cyclohexen-1-ol in DME mit 4-tert.-Butylbenzylbromid analog Beispiel 3A wurde das oben genannte Produkt in 97%iger Ausbeute erhalten.

### B. Herstellung von (1R,3S,6S)-3-(4-tert.-Butylbenzyloxy)-7-oxa-1,5,5,6-tetramethyl-bicyclo[4.1.0]heptan

Durch Umsetzen des gemäß Beispiel 5A erhaltenen (1S)-1-(4-tert.-Butylbenzyl-oxy)-3,4,5,5-tetramethyl-3-cyclohexens mit MCPBA analog Beispiel 3B wurde das oben genannte Produkt mit 92 %iger Ausbeute erhalten.

### C. Herstellung von (1R,4S)-4-Hydroxy-1,2,2-trimethyl-cyclopentylmethylketon (Ia)

Aus dem gemäß Beispiel 5B erhaltenen Epoxid wurde analog Beispiel 3C durch Umsetzen mit BF3etherat in Methylenchlorid Ia mit einem Diastereomerenüberschuß von 78 % erhalten. Die Diastereoisomeren sind destillativ oder durch Chromatographie trennbar.

### Beispiel 6

### Herstellung von (1S,4S)-4-Hydroxy-1,2,2-trimethyl-cyclopentylmethylketon (Ib)

### A. Herstellung von (1S)-1-Ethoxy-ethoxy-3,4,5,5-tetramethyl-3-cyclohexen

0,8 g (5,2 mmol) (1S)-3,4,5,5-Tetramethyl-3-cyclohexen-1-ol wurden unter N₂-Schutz in 5 ml absolutem Methylenchlorid gelöst, die Lösung auf 0°C abgekühlt und mit 260 mg (1,04 mmol) Pyridinium-p-Toluolsulfonat und danach mit 0,45 mg (6,2 mmol) Ethylvinylether versetzt. Anschließend wurde das Reaktionsgemisch 1 h bei 0°C gerührt, dann in eine gesättigte wäßrige NaHCO₃-Lösung eingetragen, mit Methylenchlorid extrahiert und der Extrakt über MgSO₄ getrocknet und eingeengt. Der Rückstand wurde durch Destillation gereinigt und ergab 1,1 g (entsprechend 97 % der Theorie) des oben genannten Produkts.

### B. Herstellung von (1R,3S,6S)-3-(Ethoxyethoxy)-7-oxa-1,5,5,6-tetramethylbicyclo[4.1.0]heptan

0,6 g (2,65 mmol) (1S)-1-Ethoxy-ethoxy-3,4,5,5-tetramethyl-3-cyclohexen wurden in 12 ml Methylenchlorid gelöst und die Lösung auf 0°C abgekühlt, dann mit 0,83 g einer 55 %igen Lösung von 3-Chlor-perbenzoesäure in Methylenchlorid (entsprechend 2,65 mmol) versetzt und anschließend 1 h bei 0°C gerührt. Durch Aufarbeitung analog Beispiel 2D wurden 0,9 g (82 % d. Theorie) erhalten. Der Diastereomerenüberschuß betrug ca. 60 %.

### C. Herstellung von (1S,4S)-4-Hydroxy-1,2,2-trimethyl-cyclopentylmethylketon

1,2 g (4,95 mmol) (1S,3S,6R)-3-(Ethoxyethoxy)-7-oxa-1,5,5,6-tetramethylbicyclo-[4.1.0]heptan wurden in 25 ml Methylenchlorid gelöst und bei RT mit 0,3 ml (2,48 mmol) Bortrifluorid-Etherat versetzt. Nach 1 h Rühren bei RT wurde der Ansatz auf 25 ml Wasser gegeben und mit 25 ml Methylenchlorid extrahiert. Zur Abspaltung der Schutzgruppe wurden der organischen Phase 2 ml einer 0,1 M Schwefelsäure zugesetzt und der Ansatz für 1 h bei RT gerührt. Anschließend wurde die organische Phase mehrfach mit Wasser gewaschen, über Magnesiumsulfat getrocknet und vom Lösungmittel befreit. Nach Chromatographie mit Hexan/ESE (3:1) wurden 0,39 g Produkt erhalten. Der Diastereomerenüberschußt betrug 60 %.

### Beispiel 7

### A. Herstellung von (1S)-1-Tetrahydropyranyloxy-3,4,5,5-tetramethyl-3-cyclohexen

0,8 g (5,2 mmol) eines gemäß Beispiel 2A hergestellten (1S)-3,4,5,5-Tetramethyl-3-cyclohexen-1-ol wurden unter N₂-Schutz in 5 ml absolutem Methylenchlorid gelöst, die Lösung auf 0°C abgekühlt und dann zunächst mit 260 mg (1,04 mmol) Pyridinium-p-Toluolsulfonat und dann mit 450 mg (6,2 mmol)3,4-Dihydropyran versetzt. Anschließend wurde das Reaktionsgemisch 1 h bei 0°C gerührt, dann in gesättigte NaHCO₃-Lösung eingegossen und mit Methylenchlorid extrahiert. Der Extrakt wurde über MgSO₄ getrocknet und eingeengt. Man erhielt 1,1 g (entsprechend 89 % d.Th.) an dem gewünschten Produkt.

Diese Verbindung kann analog Beispiel 6B und 6C in (1S,4S)-4-Hydroxy-1,2,2-trimethyl-cyclopentylmethylketon überführt werden.

### Beispiel 8

### Herstellung von (1S)-1-Benzyloxycarbonyl-3,4,5,5-tetramethyl-3-cyclohexen

2 g (13 mmol) (1S)-3,4,5,5-Tetramethyl-3-cyclohexen-1-ol wurden in 20 ml abs. Methylenchlorid gelöst. Bei RT wurde 1 ml (13 mmol) Pyridin und 1,5 ml (13 mmol) Chlorameisensäurebenzylester zugegeben. Man ließ 4 h bei RT rühren und gab das Reaktionsgemisch dann auf 100 ml Eiswasser. Nach Extraktion mit Methylenchlorid wurde die organische Phase mit 50 ml 0,1 M Schwefelsäure, 50 ml gesättigter Natriumhydrogencarbonatlösung und 50 ml Wasser gewaschen. Nach Trocknung und Entfernung des Lösungsmittels wurden 2,8 g (98 %) des oben genannten Produktes erhalten.

Durch Epoxidieren und Behandeln mit BF3etherat kann diese Verbindung analog den Beispielen 6B und 6C in (1S, 4S)-4-Hydroxy-1,2,2-trimethylcyclopentylmethylketon (Ib) überführt werden.

### Beispiel 9

### Herstellung von (1R,4R)-4-Hydroxy-1,2,2-trimethyl-cyclopentylmethylketon (Ic)

### A Herstellung von (4S)-1,4-Dihydroxy-1,2,2,6-tetramethylcyclohexan

Diese Verbindung kann analog Beispiel 1A unter Verwendung von (4S,6R)-4-Hydroxy-2,6,6-trimethyl-cyclohexan-1-on als Edukt gewonnen werden.

### B Herstellung von (1R)-3,4,5,5-Tetramethyl-3-cyclohexen-l-ol

Diese Verbindung kann aus der von Beispiel 9A analog zu Beispiel 1B erhalten werden.

### C Herstellung von (1R,3R,6S) 3-Hydroxy-7-Oxa-1,5,5,6-tetramethyl-bicyclo[4.1.0]heptan

Die Synthese erfolgt in Analogie zu Beispiel 1C und liefert in vergleichbarer Ausbeute die oben genannte Verbindung.

### D Herstellung von (1R,4R)-4-Hydroxy-1,2,2-trimethyl-cyclopentylmethylketon

In Analogie zu Beispiel 1D führte die Umsetzung von (1R,3R,6S)-3-Hydroxy-7-oxa-1,5,5,6-tetramethyl-bicyclo[4.1.0]heptan in vergleichbaren Ausbeuten und Diastereoselektivitäten zu der oben genannten Verbindung.

## Patentansprüche

1. Verfahren zur Herstellung von 1,2,2-Trimethyl-cyclopentylmethylketon-derivaten der allgemeinen Formel I 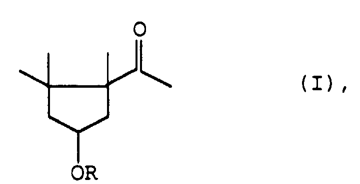 in der R für Wasserstoff oder für einen Alkyl-, Aryl-, Arylmethyl-, Trialkylsilyl-, Triarylsilyl-, Alkylarylsilyl, Alkyloxyalkyl-, Tetrahydropyranyl-, Arylmethyloxycarbonyl-, Alkanoyl- oder Benzoyl-Rest steht, dadurch gekennzeichnet, daß man
A. ein 2,2,6-Trimethyl-cyclohexan-1-on der allgemeinen Formel II 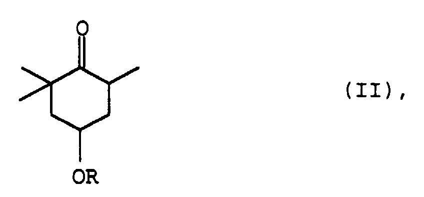 in der R die oben angegebene Bedeutung hat, mit einem Methylcarbanion der allgemeinen Formel III
CH₃⁻M⁺ (III),
in der M⁺ für Li, MgCl, MgBr oder MgJ steht, umsetzt
und das erhaltene 1,4-Dihydroxy-1,2,2,6-tetramethylcyclohexan-derivat der allgemeinen Formel IV 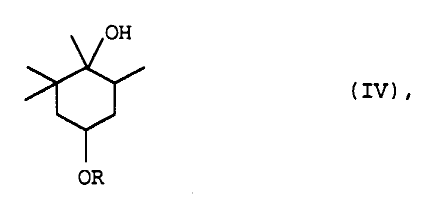 in der R die oben angegebene Bedeutung hat, durch säurekatalysierte Eliminierung der tertiären Hydroxylgruppe in ein Gemisch der entsprechenden Verbindungen der Formeln V und VI 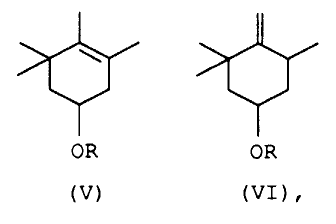 in denen R die oben angegebene Bedeutung hat, überführt, oder aber das 2,2,6-Trimethyl-cyclohexan-l-on der allgemeinen Formel II in einer Wittig-Reaktion mit einem Triphenylmethylenphosphoran der Formel VII
(C₆H₅)₃P = CH₂ (VII)
direkt in die Verbindung der allgemeinen Formel VI überführt,
B. die erhaltene Verbindung der allgemeinen Formel VI durch säurekatalysierte Doppelbindungsisomerisierung in die Verbindung der allgemeinen Formel V überführt,
C. die Doppelbindung in der Verbindung der allgemeinen Formel V 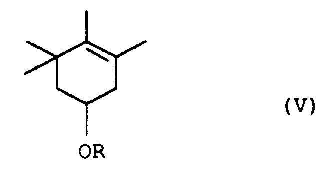 durch Umsetzen mit Peroxysäuren, deren Salzen oder Hydroperoxiden in an sich bekannter Weise epoxidiert und
D. die erhaltenen 7-Oxa-bicyclo[4.1.0]heptane der allgemeinen Formel VIII 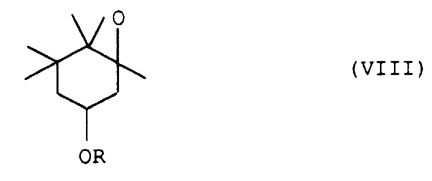 durch Umsetzen mit Lewis-Säuren und ggf. Abspalten der Schutzgruppe am Sauerstoff in die 1,2,2-Trimethyl-cyclopentylmethylketone der allgemeinen Formel I überführt.

2. Verfahren gemäß Anspruch 1, dadurch gekennzeichnet, daß man zur Herstellung von (1R, 4S)-1,2,2-Trimethyl-cyclopentylmethylketon-derivaten der allgemeinen Formel Ia 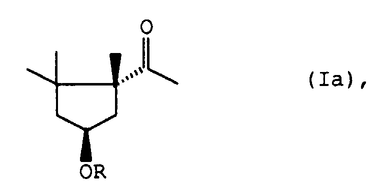 in der R für Wasserstoff oder einen tert.-Butyl-, Isopropyl-, Benzyl-, Alkylbenzyl-, 2-Naphthylmethyl-, Trialkylsilyl-, Triarylsilyl- oder Alkylarylsilyl-Rest steht, im Reaktionsschritt C. ein (1S)-3,4,5,5-Tetramethyl-3-cyclohexenderivat der allgemeinen Formel Va 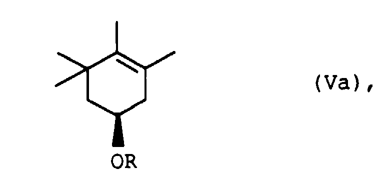 in der R für einen tert.-Butyl-, Isopropyl-, Benzyl-, Alkylbenzyl-, 2-Naphthylmethyl-, Trialkylsilyl-, Triarylsilyloder Alkylarylsilyl-Rest steht, epoxidiert und in Reaktionsschritt
D. das so erhaltene (1R, 3S, 6S)-7-Oxa-bicyclo[4.1.0]heptan der allgemeinen Formel VIIIa 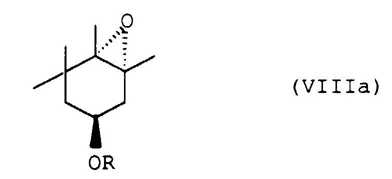 mit einer Lewis-Säure umsetzt und gewünschtenfalls die Schutzgruppe in an sich bekannter Weise abspaltet.

3. Verfahren gemäß Anspruch 1, dadurch gekennzeichnet, daß man zur Herstellung eines ggf. mit einer Schutzgruppe versehenen (1R, 4S)-4-Hydroxy-1,2,2-trimethyl-cyclopentylmethylketons der allgemeinen Formel Ia 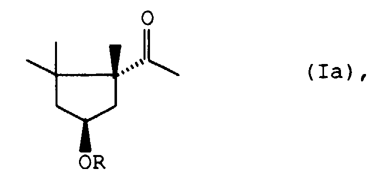 in der R für Wasserstoff, einen tert.-Butyl-, Benzyl-, Alkylbenzyl-, 2-Naphthylmethyl-, oder tert.-Butyldimethylsilylrest steht, im Reaktionsschritt
A. ein (4R, 6R)-2,2,6-Trimethyl-cyclohexan-1-on-derivat der allgemeinen Formel IIa 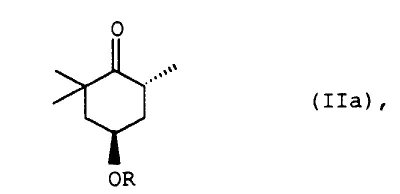 in der R für Wasserstoff steht, einsetzt, dieses gemäß den Reaktionsschritten A. und B. von Anspruch 1 in das (1S)-3,4,5,5-Tetramethyl-3-cyclohexen-1-ol der allgemeinen Formel Va 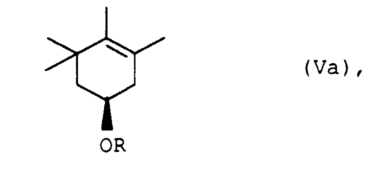 in der R für Wasserstoff steht, überführt und anschließend in an sich bekannter Weise die Hydroxylgruppe mit einer der oben genannten voluminösen, nicht koordinierenden und komplexierenden Schutzgruppen versieht,
C. das so erhaltene (1S)-3,4,5,5-Tetramethyl-3-cyclo-hexen-1-ol-derivat der allgemeinen Formel Va 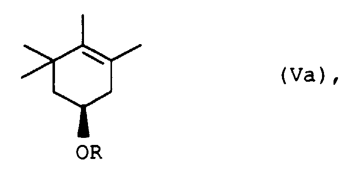 in der R für einen tert.-Butyl-, Isopropyl-, Benzyl-, Alkylbenzyl-, 2-Naphthylmethyl-, tert.-Butyl-dimethylsilylrest steht, stereoselektiv epoxidiert und
D. das so erhaltene (1R,35,65)-7-Oxa-bicyclo[4.1.0]heptan-derivat der allgemeinen Formel VIIIa 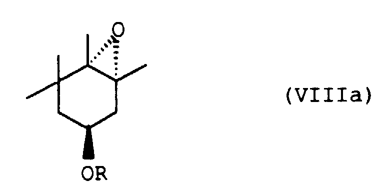 in der R die unter C. für Verbindung Va angegebene Bedeutung hat mit einer Lewis-Säure umsetzt und gewünschtenfalls die Schutzgruppe in an sich bekannter Weise entfernt.

4. Verfahren gemäß Anspruch 1, dadurch gekennzeichnet, daß man zur Herstellung von einem (1S,4S)-1,2,2-Trimethyl-cyclopentylmethylketon-derivat der allgemeinen Formel Ib 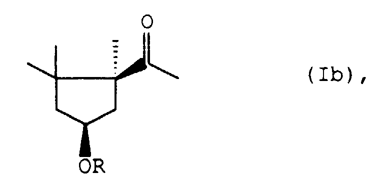 in der R für Wasserstoff oder für einen Benzyloxycarbonyl-, Tetrahydropyranyl-, Ethoxyethyl-, Acetyl- oder Methoxyisopropyl-Rest steht, im Reaktionsschritt C. ein (1S)-3,4,5,5-Tetramethyl-3-cyclohexen-derivat der allgemeinen Formel Va 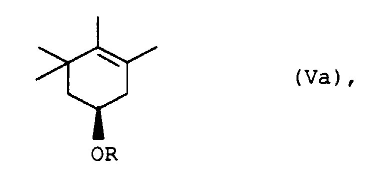 in der R für Wasserstoff oder einen der für Ib genannten Reste steht, diastereoselektiv epoxidiert,
D. das erhaltene (1S,3S,6R)-7-Oxa-1,5,5,6-tetramethyl-bicyclo[4.1.0]heptan-derivat der allgemeinen Formel VIIIb 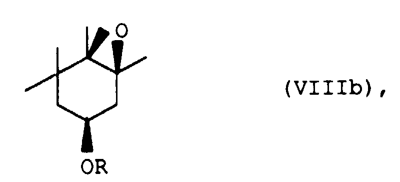 in der R für Wasserstoff oder einen der oben für Ib genannten Reste steht, mit einer Lewissäure umsetzt und gewünschtenfalls die Schutzgruppe in an sich bekannter Weise entfernt.

5. Verfahren gemäß Anspruch 1, dadurch gekennzeichnet, daß man zur Herstellung von einem (1S,45)-1,2,2-Trimethyl-cyclopentylmethylketon der allgemeinen Formel Ib 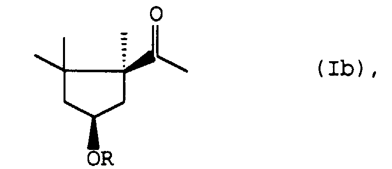 in der R für Wasserstoff oder einen Benzyloxycarbonyl-, Tetrahydropyranyl-, Ethoxyethyl-, Acetyl- oder Methoxyisopropyl-Rest steht, ein (4R,6R)-2,2,6-Trimethyl-cyclohexan-1-on-derivat der allgemeinen Formel IIa 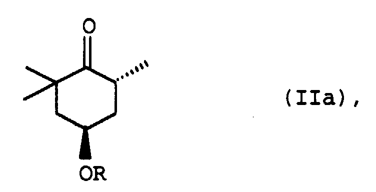 in der R für H oder einen Benzyloxycarbonyl-, Tetrahydropyranyl-, Ethoxyethyl-, Acetyl- oder Methoxyisopropylrest steht, einsetzt, dieses gemäß den beschriebenen Reaktionsschritten A. und B. von Anspruch 1 in ein (1S)-3,4,5,5-Tetramethyl-3-cyclohexen-derivat der allgemeinen Formel Va 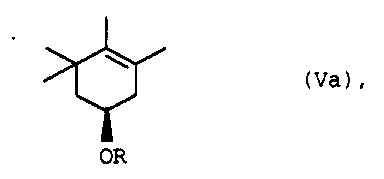 in der R für Wasserstoff oder einen Benzyloxycarbonyl-, Tetrahydropyranyl-, Ethoxyethyl-, Acetyl- oder Methoxyisopropylrest steht, überführt,
C. dieses stereoselektiv epoxidiert und
D. das so erhaltene (1S,3S,6R)-7-Oxa-1,5,5,6-tetramethyl-bicyclo[4.1.0]heptan-derivat der allgemeinen Formel VIIIb 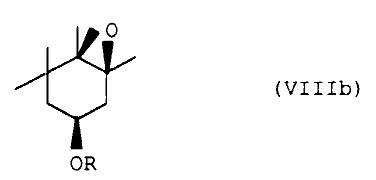 mit einer Lewis-Säure umsetzt und gewünschtenfalls die Schutzgruppe in an sich bekannter Weise entfernt.

6. Verfahren gemäß Anspruch 1, dadurch gekennzeichnet, daß man zur Herstellung von einem (1R,4R)-1,2,2-Trimethyl-cyclopentylmethylketon-derivat der Formel Ic 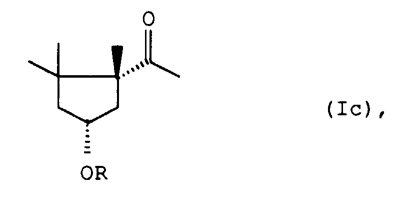 in der R für Wasserstoff oder einen Benzyloxycarbonyl-, Tetrahydropyranyl-, Ethoxyethyl-, Acetyl- oder Methoxyisopropylrest steht,
im Reaktionsschritt C. ein (1R)-3,4,5,5-Tetramethyl-3-cyclohexen-derivat der allgemeinen Formel Vb 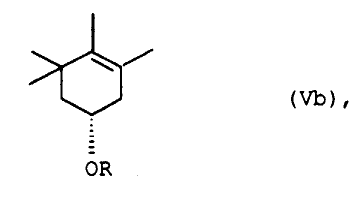 in der R für Wasserstoff oder einen der oben für Ic genannten Reste steht, stereoselektiv epoxidiert und
D. das erhaltene (1R,3R,6S)-7-Oxa-1,5,5,6-tetramethyl-bicyclo[4.1.0]heptan-derivat der allgemeinen Formel VIIIc 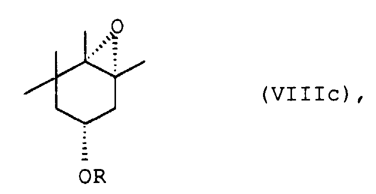 mit einer Lewis-Säure umsetzt und gewünschtenfalls die Schutzgruppe in an sich bekannter Weise entfernt.

7. Verfahren gemäß Anspruch 1, dadurch gekennzeichnet, daß man zur Herstellung von (1R,4R)-4-Hydroxy-1,2,2-trimethyl-cyclopentylmethylketon der allgemeinen Formel Ic 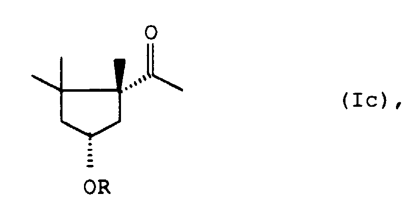 in der R für H steht, im Reaktionsschritt C. ein (1R)-3,4,5,5-Tetramethyl-3-cyclohexen-Derivat der allgemeinen Formel Vb 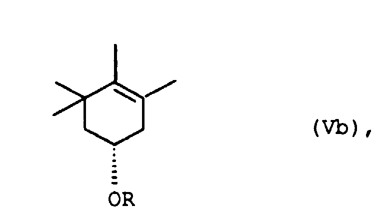 in der R für Wasserstoff oder einen Benzyloxycarbonyl-, Tetrahydropyranyl-, Ethoxyethyl- oder Methoxyisopropyl-rest steht, stereoselektiv epoxidiert und im Reaktionsschritt
D. das so erhaltene (1R, 3R, 6S)-7-Oxa-1,5,5,6-tetramethylbicyclo[4.1.0]heptan-derivat der allgemeinen Formel VIIIc 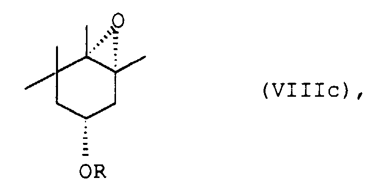 mit einer Lewis-Säure umsetzt und gewünschtenfalls die Schutzgruppe in an sich bekannter Weise abspaltet.

8. 3,4,5,5-Tetramethyl-3-cyclohexen-derivate der allgemeinen Formel V 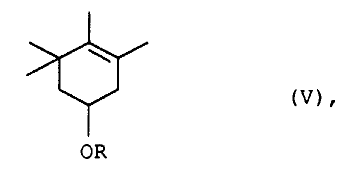 in der R für einen tert.-Butyl-, Isopropyl-, Benzyl-, Alkylbenzyl-, 2-Naphthylmethyl-, Trialkylsilyl-, Triarylsilyl-, Alkylarylsilyl-, Benzyloxycarbonyl-, Tetrahydropyranyl-, Ethoxyethyl- oder Methoxyisopropyl-Rest steht sowie deren (1S)- und (lR)-Isomeren der Formeln Va bzw. Vb 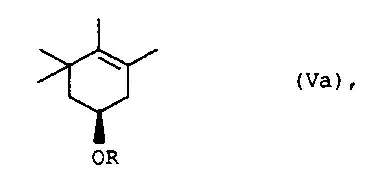 in der R für einen tert.-Butyl-, Isopropyl-, Benzyl-, Alkylbenzyl-, 2-Naphthylmethyl-, Trialkylsilyl-, Triarylsilyl- oder Alkylarylsilyl-Rest steht, 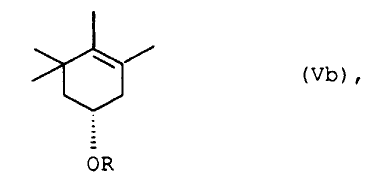 in der R für einen Benzyloxycarbonyl-, Tetrahydropyranyl-, Ethoxyethyl- oder Methoxyisopropylrest steht.

9. 7-Oxa-1,5,5,6-tetramethyl-bicyclo[4.1.0]heptan-derivate der allgemeinen Formel VIII 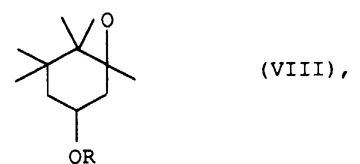 in der R für einen tert.-Butyl-, Isopropyl-, Benzyl-, Alkylbenzyl-, 2-Naphthylmethyl-, Trialkylsilyl-, Triarylsilyl-, Alkylarylsilyl-, Benzyloxycarbonyl-, Tetrahydropyranyl-, Ethoxyethyl- oder Methoxyisopropyl-Rest steht sowie deren (1R,3S,6S)-Isomeren der allgemeinen Formel VIIIa 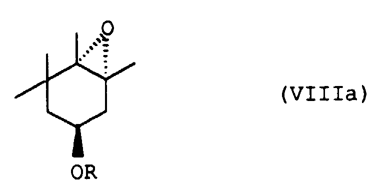 in der R für einen tert.-Butyl-, Isopropyl-, Benzyl-, Alkylbenzyl-, 2-Naphthylmethyl- oder tert.-Butyldimethylsilyl-Rest steht;
(1S,3S,6R)-Isomeren der allgemeinen Formel VIIIb 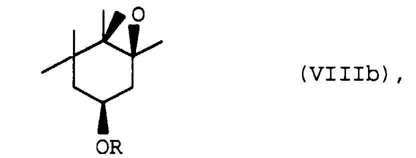 in der R für einen Benzyloxycarbonyl-, Tetrahydropyranyl-, Ethoxyethyl- oder Methoxyisopropyl-Rest steht,
und (1R,3R,6S)-Isomeren der allgemeinen Formel VIIIc 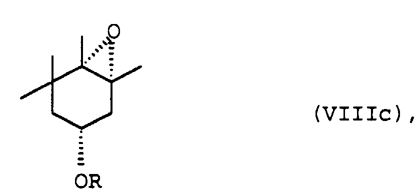 in der R für einen Benzyloxycarbonyl-, Tetrahydropyranyl-, Ethoxyethyl- oder Methoxyisopropyl-Rest steht.

10. (1S)-3,4,5,5-Tetramethyl-3-cyclohexen-derivate der allgemeinen Formel Va 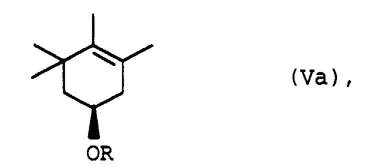 in der R für einen tert.-Butyl-, Isopropyl-, Benzyl-, Alkylbenzyl-, 2-Napthylmethyl- oder tert.-Butyldimethylsilyl-Rest steht.

11. (1R,3S,6S)-7-Oxa-1,5,5,6-tetramethyl-bicyclo[4.1.0]heptan-derivate der allgemeinen Formel VIIIa 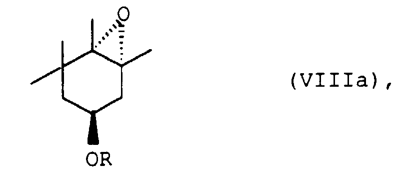 in der R für einen tert.-Butyl-, Isopropyl-, Benzyl-, Alkylbenzyl-, 2-Naphthylmethyl- oder tert.-Butyldimethylsilyl-Rest steht.

## Claims

1. A process for preparing 1,2,2-trimethylcyclopentyl methyl ketone derivatives of the general formula I 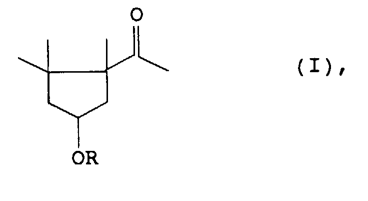 where R is hydrogen or alkyl, aryl, arylmethyl, trialkylsilyl, triarylsilyl, alkylarylsilyl, alkoxyalkyl, tetrahydropyranyl, arylmethyloxycarbonyl, alkanoyl or benzoyl, which comprises
A. reacting a 2,2,6-trimethyl-1-cyclohexanone of the general formula II 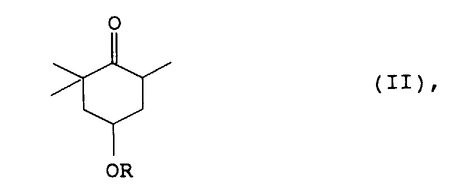 where R has the abovementioned meanings, with a methyl carbanion of the general formula III
CH₃⁻M⁺ (III),
where M⁺ is Li, MgCl, MgBr or MgI,
and converting the resulting 1,4-dihydroxy-1,2,2,6-tetramethylcyclohexane derivative of the general formula IV 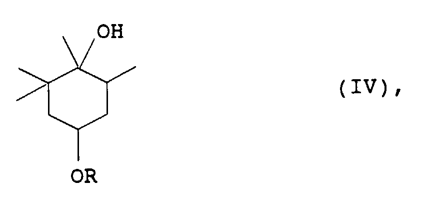 where R has the abovementioned meanings, by acid-catalyzed elimination of the tertiary hydroxyl group into a mixture of compounds of the formulae V and VI 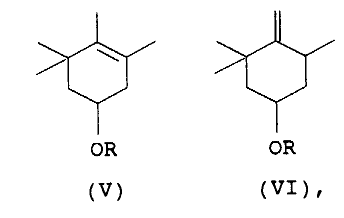 where R has the abovementioned meanings, or else converting the 2,2,6-trimethyl-1-cyclohexanone of the general formula II in a Wittig reaction with a methylenetriphenylphosphorane of the formula VII
(C₆H₅)₃P = CH₂ (VII)
directly into the compound of the general formula VI,
B. converting the resulting compound of the general formula VI by acid-catalyzed double-bond isomerization into the compound of the general formula V,
C. epoxidizing the double bond in the compound of the general formula V 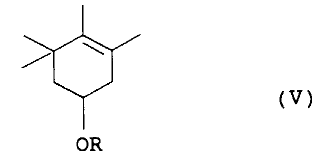 by reaction with peroxy acids, their salts or hydroperoxides in a conventional way, and
D. converting the resulting 7-oxabicyclo[4.1.0]heptanes of the general formula VIII 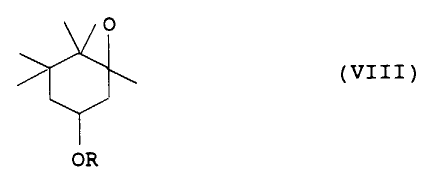 by reaction with Lewis acids and, where appropriate, elimination of the protective group on the oxygen into the 1,2,2-trimethylcyclopentyl methyl ketones of the general formula I.

2. A process as claimed in claim 1, wherein, for the preparation of (1R, 4S)-1,2,2-trimethylcyclopentyl methyl ketone derivates of the general formula Ia 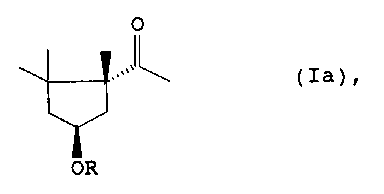 where R is hydrogen or tert-butyl, isopropyl, benzyl, alkylbenzyl, 2-naphthylmethyl, trialkylsilyl, triarylsilyl or alkylarylsilyl, in step C. a (1S)-3,4,5,5-tetramethyl-3-cyclohexene derivative of the general formula Va 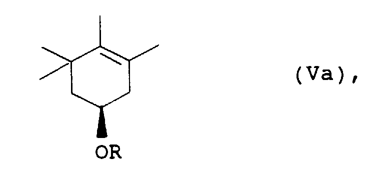 where R is tert-butyl, isopropyl, benzyl, alkylbenzyl, 2-naphthylmethyl, trialkylsilyl, triarylsilyl or alkylarylsilyl, is epoxidized, and in step
D. the resulting (1R,3S,6S)-7-oxa-bicyclo[4.1.0]heptane of the general formula VIIIa 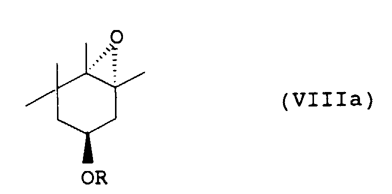 is reacted with a Lewis acid and, if required, the protective group is eliminated in a conventional way.

3. A process as claimed in claim 1, wherein, for the preparation of a (1R, 4S)-4-hydroxy-1,2,2-trimethylcyclopentyl methyl ketone, which is provided with a protective group where appropriate, of the general formula Ia 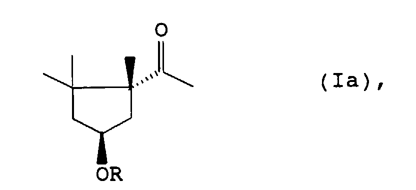 where R is hydrogen, tert-butyl, benzyl, alkylbenzyl, 2-naphthylmethyl or tert-butyldimethylsilyl, in step
A. a (4R,6R)-2,2,6-trimethyl-1-cyclohexanone derivative of the general formula IIa 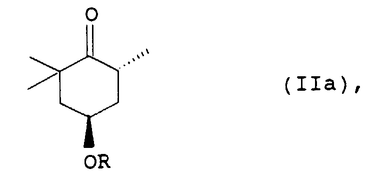 where R is hydrogen, is used and is converted as in steps A. and B. in claim 1 into the (1S)-3,4,5,5-tetramethyl-3-cyclohexen-1-ol of the general formula Va 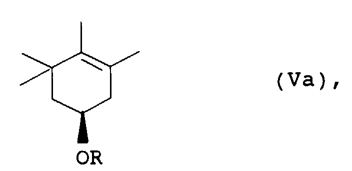 where R is hydrogen, and subsequently the hydroxyl group is provided in a conventional way with one of the abovementioned bulky, non-coordinating and complexing protective groups,
C. the resulting (1S)-3,4,5,5-tetramethyl-3-cyclohexen-1-ol derivative of the general formula Va 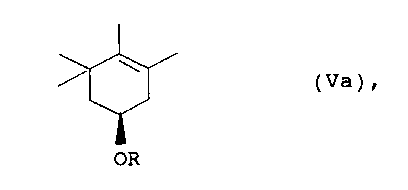 where R is tert-butyl, isopropyl, benzyl, alkylbenzyl, 2-naphthylmethyl or tert-butyldimethylsilyl, is stereoselectively epoxidized and
D. the resulting (1R,3S,6S)-7-oxabicyclo[4.1.0]heptane derivative of the general formula VIIIa 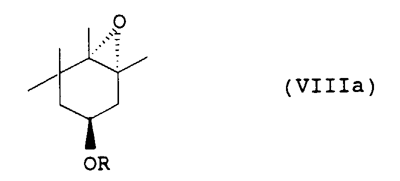 where R has the meanings stated under C. for compound Va, is reacted with a Lewis acid and, if required, the protective group is removed in a conventional way.

4. A process as claimed in claim 1, wherein, for the preparation of a (1S,4S)-1,2,2-trimethylcyclopentyl methyl ketone derivative of the general formula Ib 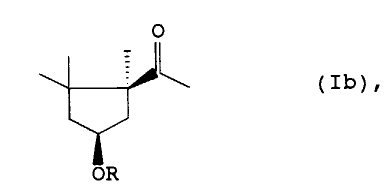 where R is hydrogen or benzyloxycarbonyl, tetrahydropyranyl, ethoxyethyl, acetyl or methoxyisopropyl, in step C. a (1S)-3,4,5,5-tetramethyl-3-cyclohexene derivative of the general formula Va 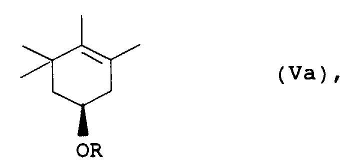 where R is hydrogen or one of the radicals mentioned for Ib, is diastereoselectively epoxidized,
D. the resulting (1S,3S,6R)-7-oxa-1,5,5,6-tetramethylbicyclo[4.1.0]heptane derivative of the general formula VIIIb 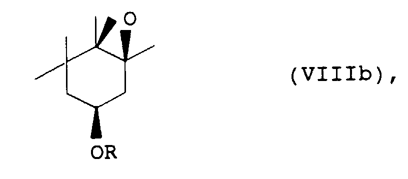 where R is hydrogen or one of the radicals mentioned above for Ib, is reacted with a Lewis acid and, if required, the protective group is removed in a conventional way.

5. A process as claimed in claim 1, wherein, for the preparation of a (1S,4S)-1,2,2-trimethylcyclopentyl methyl ketone of the general formula Ib 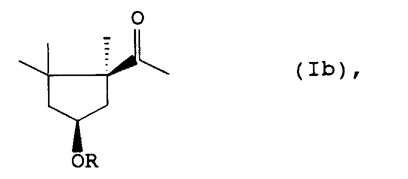 where R is hydrogen or benzyloxycarbonyl, tetrahydropyranyl, ethoxyethyl, acetyl or methoxyisopropyl, a (4R,6R)-2,2,6-trimethyl-1-cyclohexanone derivative of the general formula IIa 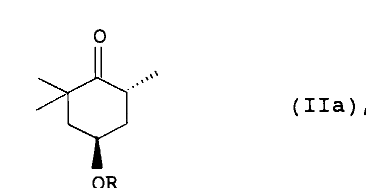 where R is H or a benzyloxycarbonyl, tetrahydropyranyl, ethoxyethyl, acetyl or methoxyisopropyl, is used and is converted as in steps A. and B. of claim 1 into a (1S)-3,4,5,5-tetramethyl-3-cyclohexene derivative of the general formula Va 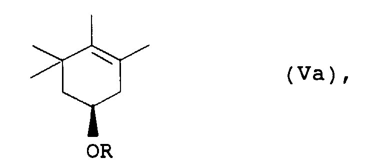 where R is hydrogen or benzyloxycarbonyl, tetrahydropyranyl, ethoxyethyl, acetyl or methoxyisopropyl,
C. the latter is stereoselectively epoxidized and
D. the resulting (1S,3S,6R)-7-oxa-1,5,5,6-tetramethyl-bicyclo[4.1.0]heptane derivative of the general formula VIIIb 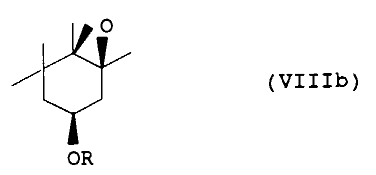 is reacted with a Lewis acid and, if required, the protective group is removed in a conventional way.

6. A process as claimed in claim 1, wherein, for the preparation of a (1R,4R)-1,2,2-trimethylcyclopentyl methyl ketone derivative of the formula Ic 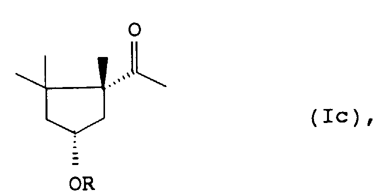 where R is hydrogen or benzyloxycarbonyl, tetrahydropyranyl, ethoxyethyl, acetyl or methoxyisopropyl,
in step C. a (1R)-3,4,5,5-tetramethyl-3-cyclohexene derivative of the general formula Vb 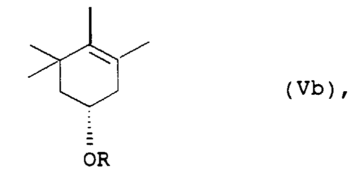 where R is hydrogen or one of the radicals mentioned above for Ic, is stereoselectively epoxidized and
D. the resulting (1R,3R,6S)-7-oxa-1,5,5,6-tetramethylbicyclo[4.1.0]heptane derivative of the general formula VIIIc 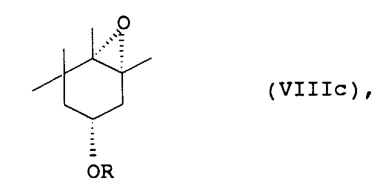 is reacted with a Lewis acid and, if required, the protective group is removed in a conventional way.

7. A process as claimed in claim 1, wherein, for the preparation of (1R,4R)-4-hydroxy-1,2,2-trimethylcyclopentyl methyl ketone of the general formula Ic 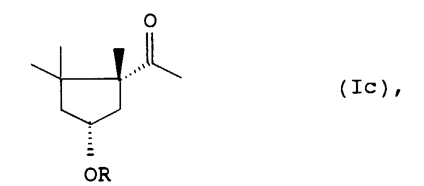 where R is H, in step C. a (1R)-3,4,5,5-tetramethyl-3-cyclohexene derivative of the general formula Vb 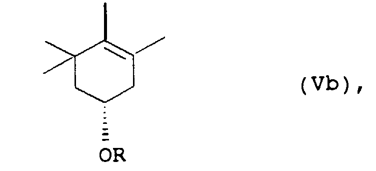 where R is hydrogen or benzyloxycarbonyl, tetrahydropyranyl, ethoxyethyl or methoxyisopropyl, is stereoselectively epoxidized, and in step
D. the resulting (1R, 3R, 6S)-7-oxa-1,5,5,6-tetramethylbicyclo[4.1.0]heptane derivative of the general formula VIIIc 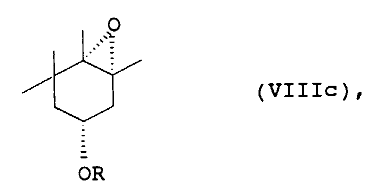 is reacted with a Lewis acid and, if required, the protective group is eliminated in a conventional way.

8. A 3,4,5,5-tetramethyl-3-cyclohexene derivative of the general formula V 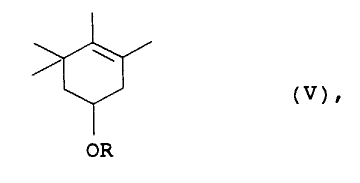 where R is tert-butyl, isopropyl, benzyl, alkylbenzyl, 2-naphthylmethyl, trialkylsilyl, triarylsilyl, alkylarylsilyl, benzyloxycarbonyl, tetrahydropyranyl, ethoxyethyl or methoxyisopropyl, and its (1S) and (1R) isomers of the formulae Va and Vb respectively 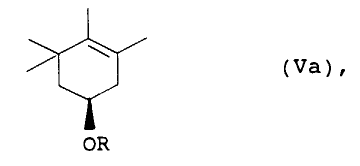 where R is tert-butyl, isopropyl, benzyl, alkylbenzyl, 2-naphthylmethyl, trialkylsilyl, triarylsilyl or alkylarylsilyl, 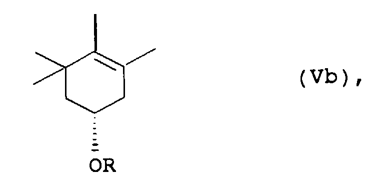 where R is benzyloxycarbonyl, tetrahydropyranyl, ethoxyethyl or methoxyisopropyl.

9. A 7-oxa-1,5,5,6-tetramethylbicyclo[4.1.0]heptane derivative of the general formula VIII 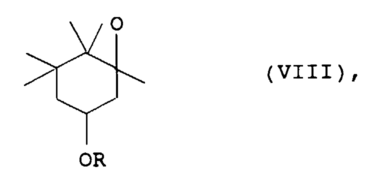 where R is tert-butyl, isopropyl, benzyl, alkylbenzyl, 2-naphthylmethyl, trialkylsilyl, triarylsilyl, alkylarylsilyl, benzyloxycarbonyl, tetrahydropyranyl, ethoxyethyl or methoxyisopropyl, and its (1R,3S,6S) isomer of the general formula VIIIa 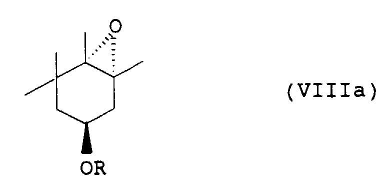 where R is tert-butyl, isopropyl, benzyl, alkylbenzyl, 2-naphthylmethyl or tert-butyldimethylsilyl; (1S,3S,6R) isomer of the general formula VIIIb 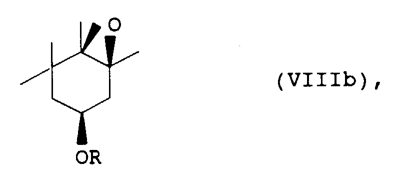 where R is benzyloxycarbonyl, tetrahydropyranyl, ethoxyethyl or methoxyisopropyl, and (1R,3R,6S) isomer of the general formula VIIIc 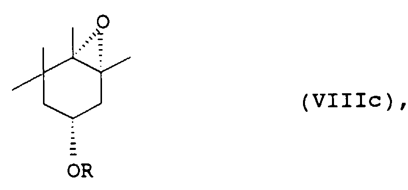 where R is benzyloxycarbonyl, tetrahydropyranyl, ethoxyethyl or methoxyisopropyl.

10. A (1S)-3,4,5,5-tetramethyl-3-cyclohexene derivative of the general formula Va 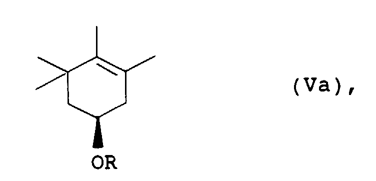 where R is tert-butyl, isopropyl, benzyl, alkylbenzyl, 2-naphthylmethyl or tert-butyldimethylsilyl.

11. A (1R,3S,6S)-7-oxa-1,5,5,6-tetramethylbicyclo[4.1.0]-heptane derivative of the general formula VIIIa 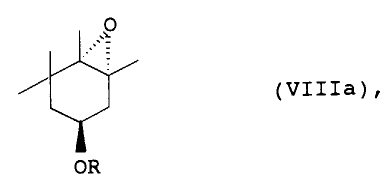 where R is tert-butyl, isopropyl, benzyl, alkylbenzyl, 2-naphthylmethyl or tert-butyldimethylsilyl.

## Revendications

1. Procédé de préparation de dérivés de la 1,2,2-triméthylcyclopentylméthylcétone de formule générale I 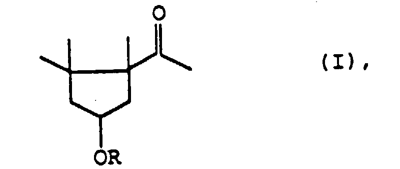 dans laquelle R représente l'hydrogène ou un groupe alkyle, aryle, arylméthyle, trialkylsilyle, triarylsilyle, alkylarylsilyle, alkyloxyalkyle, tétrahydropyrannyle, arylméthoxycarbonyle, alcanoyle ou benzoyle, caractérisé par le fait que
A. on fait réagir une 2,2,6-triméthylcyclohexane-1-one de formule générale II 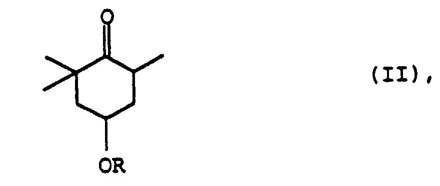 dans laquelle R a les significations indiquées ci-dessus, avec un méthylcarbanion de formule générale III
CH₃⁻M⁺ (III),
dans laquelle M⁺ représente Li, MgCl, MgBr ou MgI,
ce qui donne un dérivé de 1,4-dihydroxy-1,2,2,6-tétraméthylcyclohexane de formule générale IV 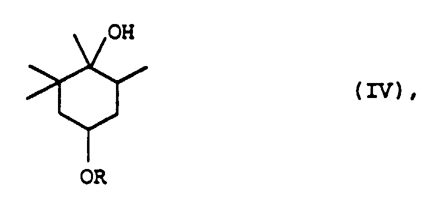 dans laquelle R a les significations indiquées ci-dessus, qu'on convertit par élimination du groupe hydroxyle tertiaire, catalysée par un acide, en un mélange des composés correspondants de formules V et VI 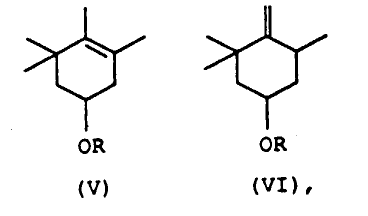 dans lesquelles R a les significations indiquées ci-dessus, ou bien on convertit directement la 2,2,6-triméthylcyclohexane-1-one de formule générale II, dans une réaction de Wittig avec un triphénylméthylènephosphorane de formule VII, en le composé de formule générale VI :
(C₆H₅)₃P = CH₂ (VII)
B : on convertit le composé obtenu, répondant à la formule générale VI, par isomérisation de la double liaison, catalysée par un acide, en le composé de formule générale V,
C. on époxyde la double liaison du composé de formule générale V 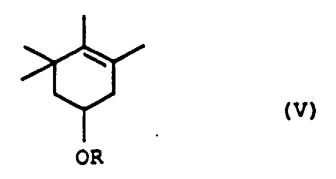 par réaction avec des peroxyacides, leurs sels ou des hydroperoxydes, de manière connue en soi, et
D. on convertit les 7-oxa-bicyclo[4.1.0]heptanes obtenus, de formule générale VIII 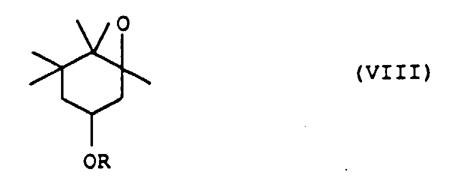 par réaction avec des acides de Lewis et le cas échéant scission du groupe protecteur sur l'oxygène, en les 1,2,2-triméthylcyclopentylcétones de formule générale I.

2. Procédé selon la revendication 1, caractérisé par le fait que, pour la préparation de dérivés de (1R,4S)-1,2,2-triméthylcyclopentylméthylcétone de formule générale Ia 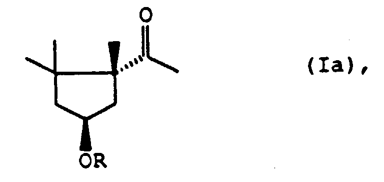 dans laquelle R représente l'hydrogène ou un groupe tert-butyle, isopropyle, benzyle, alkylbenzyle, 2-naphtylméthyle, trialkylsilyle, triarylsilyle ou alkylarylsilyle, on époxyde dans le stade de réaction C, un dérivé de (1S)-3,4,5,5-tétraméthyl-3-cyclohexène de formule générale Va 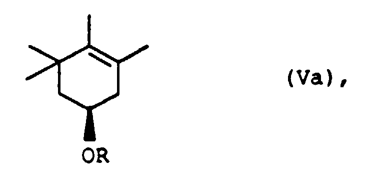 dans laquelle R représente un groupe tert-butyle, iso-propyle, benzyle, alkylbenzyle, 2-naphtylméthyle, trialkylsilyle, triarylsilyle ou alkylarylsilyle, et dans le stade de réaction
D. on fait réagir le (1R,3S,6S)-7-oxa-bicyclo[4.1.0]heptane obtenu, de formule générale VIIIa 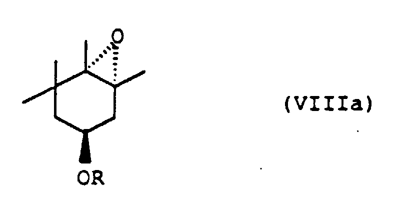 avec un acide de Lewis et, si on le désire, on scinde le groupe protecteur de manière connue en soi.

3. Procédé selon la revendication 1, caractérisé par le fait que, pour la préparation d'une (1R,4S)-4-hydroxy-1,2,2-triméthylcyclopentylméthylcétone, portant éventuellement un groupe protecteur, de formule générale Ia 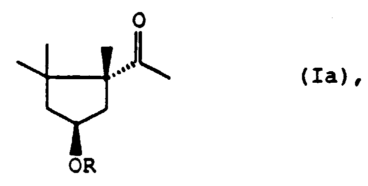 dans laquelle R représente l'hydrogène, un groupe tert-butyle, benzyle, alkylbenzyle, 2-naphtylméthyle ou tert-butyldiméthylsilyle, au stade de réaction
A. on met en oeuvre un dérivé de (4R,6R)-2,2,6-triméthylcyclohexane-1-one de formule générale IIa 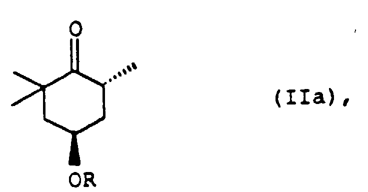 dans laquelle R représente l'hydrogène, on convertit ce dérivé dans les stades de réaction A. et B. de la revendication 1, en le (1S)-3,4,5,5-tétraméthyl-3-cyclohexène-1-ol de formule générale Va 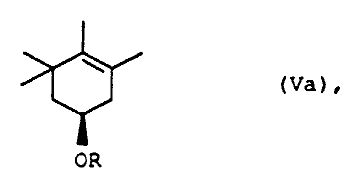 dans laquelle R représente l'hydrogène, puis on protège de manière connue en soi le groupe hydroxyle par l'un des groupes protecteurs volumineux, non coordinants et complexants mentionnés ci-dessus,
C. ce qui donne un dérivé de (1S)-3,4,5,5-tétraméthyl-3-cyclohexène-1-ol de formule générale Va 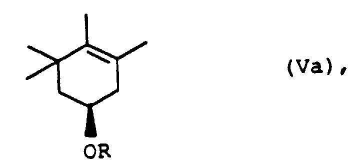 dans laquelle R représente un groupe tert-butyle, iso-propyle, benzyle, alkylbenzyle, 2-naphtylméthyle, tert-butyl-diméthylsilyle, qu'on soumet à époxydation stéréosélective,
D. ce qui donne un dérivé de (1R,3S,6S)-7-oxa-bicyclo[4.1.0]heptane de formule générale VIIIa 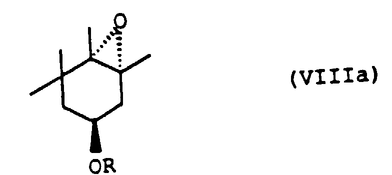 dans laquelle R a les significations indiquées en C. pour le composé Va, qu'on fait réagir avec un acide de Lewis, après quoi, si on le désire, on élimine le groupe protecteur de manière connue en soi.

4. Procédé selon la revendication 1, caractérisé par le fait que, pour la préparation d'un dérivé de (1S,4S)-1,2,2-triméthylcyclopentylméthylcétone de formule générale Ib 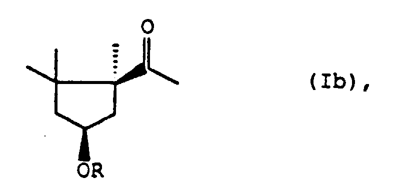 dans laquelle R représente l'hydrogène ou un groupe benzyloxycarbonyle, tétrahydropyrannyle, éthoxyéthyle, acétyle ou méthoxyisopropyle, on soumet à époxydation diastéréosélective au stade de réaction C. un dérivé de (1S)-3,4,5,5-tétraméthyl-3-cyclohexène de formule générale Va 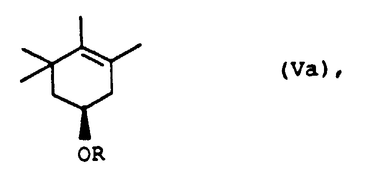 dans laquelle R représente l'hydrogène ou l'un des substituants mentionnés en référence à Ib,
D. ce qui donne un dérivé de (1S,3S,6R)-7-oxa-1,5,5,6-tétraméthylbicyclo[4.1.0]heptane de formule générale VIIIb 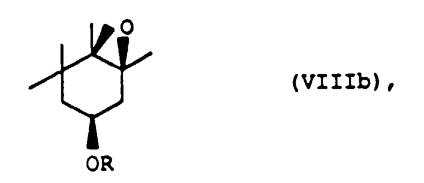 dans laquelle R représente l'hydrogène ou l'un des substituants mentionnés ci-dessus en référence à Ib, qu'on fait réagir avec un acide de Lewis, après quoi, si on le désire, on élimine le groupe protecteur de manière connue en soi.

5. Procédé selon la revendication 1, caractérisé par le fait que, pour la préparation d'une (1S,4S)-1,2,2-triméthylcyclopentylméthylcétone de formule générale Ib 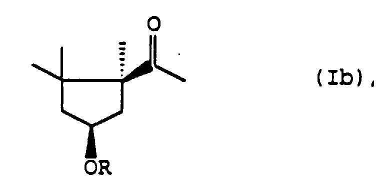 dans laquelle R représente l'hydrogène ou un groupe benzyloxycarbonyle, tétrahydropyrannyle, éthoxyéthyle, acétyle ou méthoxyisopropyle, on met en oeuvre un dérivé de (4R,6R)-2,2,6-triméthylcyclohexane-1-one de formule générale IIa 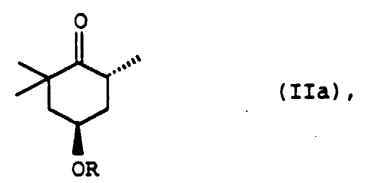 dans laquelle R représente H ou un groupe benzyloxycarbonyle, tétrahydropyrannyle, éthoxyéthyle, acétyle ou méthoxyisopropyle, on le convertit comme décrit dans les stades de réaction A. et B. de la revendication 1, en un dérivé de (1S)-3,4,5,5-tétraméthyl-3-cyclohexène de formule générale Va 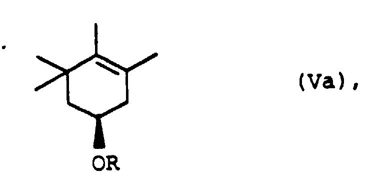 dans laquelle R représente l'hydrogène ou un groupe benzyloxycarbonyle, tétrahydropyrannyle, éthoxyéthyle, acétyle ou méthoxyisopropyle,
C. qu'on soumet à époxydation stéréosélective,
D. ce qui donne un dérivé de (1S,3S,6R)-7-oxa-1,5,5,6-tétraméthyl-bicyclo[4.1.0]heptane de formule générale VIIIb 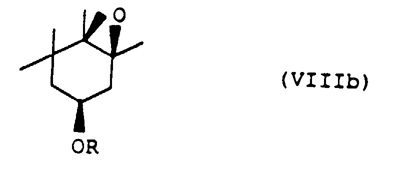 qu'on fait réagir avec un acide de Lewis, après quoi, si on le désire, on élimine le groupe protecteur de manière connue en soi.

6. Procédé selon la revendication 1, caractérisé par le fait que, pour la préparation d'un dérivé de (1R,4R)-1,2,2-triméthylcyclopentylméthylcétone de formule Ic 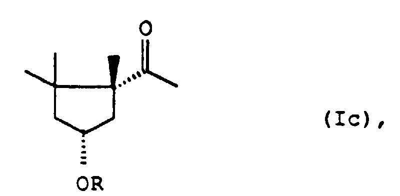 dans laquelle R représente l'hydrogène ou un groupe benzyloxycarbonyle, tétrahydropyrannyle, éthoxyéthyle, acétyle ou méthoxyisopropyle,
on soumet au stade de réaction C. un dérivé de (1R)-3,4,5,5-tétraméthyl-3-cyclohexène de formule générale Vb 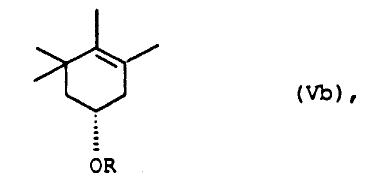 dans laquelle R représente l'hydrogène ou l'un des substituants mentionnés ci-dessus en référence à Ic, à époxydation stéréosélective,
D. ce qui donne un dérivé de (1R,3R,6S)-7-oxa-1,5,5,6-tétraméthylbicyclo[4.1.0]heptane de formule générale VIIIc 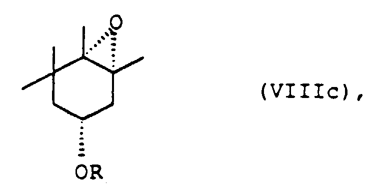 qu'on fait réagir avec un acide de Lewis après quoi, si on le désire, on élimine le groupe protecteur de manière connue en soi.

7. Procédé selon la revendication 1, caractérisé par le fait que, pour la préparation d'une (1R,4R)-4-hydroxy-1,2,2-triméthylcyclopentylméthylcétone de formule générale Ic 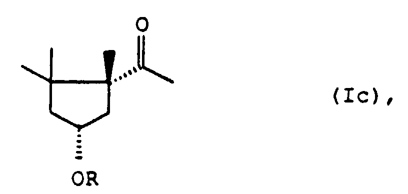 dans laquelle R représente H, on soumet à époxydation stéréosélective au stade de réaction C. un dérivé de (lR)-3,4,5,5-tétraméthyl-3-cyclohexène de formule générale Vb 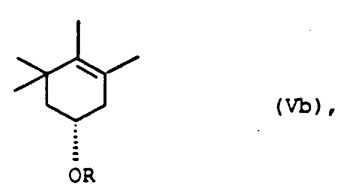 dans laquelle R représente l'hydrogène ou un groupe benzyloxycarbonyle, tétrahydropyrannyle, éthoxyéthyle ou méthoxyisopropyle, ce qui donne
D. un dérivé de (1R,3R,6S)-7-oxa-1,5,5,6-tétraméthylbicyclo[4.1.0]heptane de formule générale VIIIc 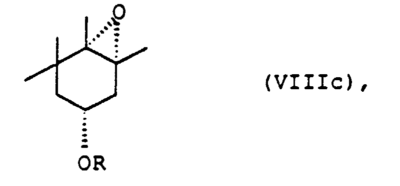 qu'on fait réagir avec un acide de Lewis après quoi, si on le désire, on élimine le groupe protecteur de manière connue en soi.

8. Dérivés du 3,4,5,5-tétraméthyl-3-cyclohexène de formule générale V 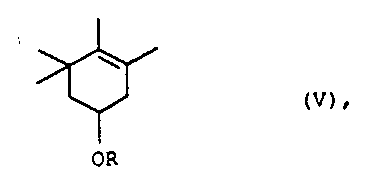 dans laquelle R représente un groupe tert-butyle, iso-propyle, benzyle, alkylbenzyle, 2-naphtylméthyle, trialkylsilyle, triarylsilyle, alkylarylsilyle, benzyloxycarbonyle, tétrahydropyrannyle, éthoxyéthyle ou méthoxyisopropyle, et leurs isomères (1S) et (1R) de formules respectives Va et Vb 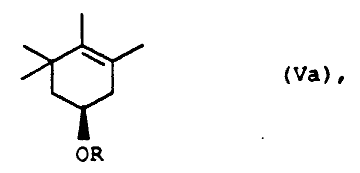 dans laquelle R représente un groupe tert-butyle, iso-propyle, benzyle, alkylbenzyle, 2-naphtylméthyle, trialkylsilyle, triarylsilyle ou alkylarylsilyle. 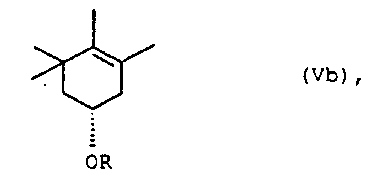 dans laquelle R représente un groupe benzyloxycarbonyle, tétrahydropyrannyle, éthoxyéthyle ou méthoxyisopropyle.

9. Dérivés du 7-oxa-1,5,5,6-tétraméthyl-bicyclo[4.1.0]heptane de formule générale VIII 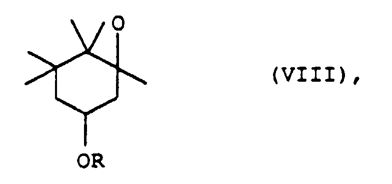 dans laquelle R représente un groupe tert-butyle, iso-propyle, benzyle, alkylbenzyle, 2-naphtylméthyle, trialkylsilyle, triarylsilyle, alkylarylsilyle, benzyloxycarbonyle, tétrahydropyrannyle, éthoxyéthyle ou méthoxyisopropyle et leurs isomères (1R,3S,6S) de formule générale VIIIa 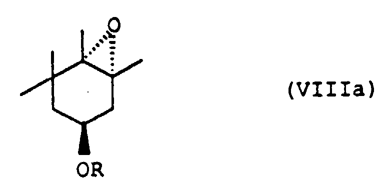 dans laquelle R représente un groupe tert-butyle, iso-propyle, benzyle, alkylbenzyle, 2-naphtylméthyle ou tert-butyldiméthylsilyle ;
leurs isomères (1S,3S,6R) de formule générale VIIIb 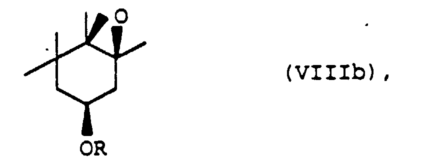 dans laquelle R représente un groupe benzyloxycarbonyle, tétrahydropyrannyle, éthoxyéthyle ou méthoxyisopropyle,
et leurs isomères (1R,3R,6S) de formule générale VIIIc 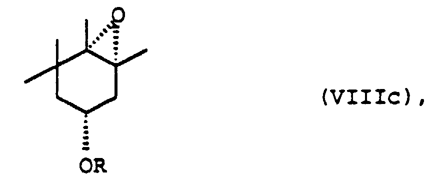 dans laquelle R représente un groupe benzyloxycarbonyle, tétrahydropyrannyle, éthoxyéthyle ou méthoxyisopropyle.

10. Dérivés du (1S)-3,4,5,5-tétraméthyl-3-cyclohexène de formule générale Va 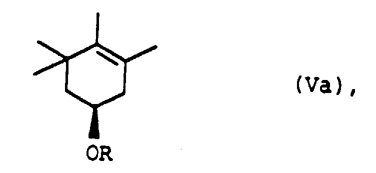 dans laquelle R représente un groupe tert-butyle, iso-propyle, benzyle, alkylbenzyle, 2-naphtylméthyle ou tert-diméthylsilyle.

11. Dérivés du (1R,3S,6S)-7-oxa-1,5,5,6-tétraméthylbicyclo[4.1.0]-heptane, de formule générale VIIIa 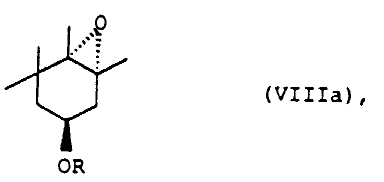 dans laquelle R représente un groupe tert-butyle, iso-propyle, benzyle, alkylbenzyle, 2-naphtylméthyle ou tert-butyldiméthylsilyle.
